(19) Europäisches Patentamt
European Patent Office
Office européen des brevets

(11) Veröffentlichungsnummer: **0 024 665**
**B1**

(12) # EUROPÄISCHE PATENTSCHRIFT

(45) Veröffentlichungstag der Patentschrift: **05.05.82**

(51) Int. Cl.³: **C 07 C 119/048**, C 07 C 118/00, C 08 G 18/76

(21) Anmeldenummer: **80104883.6**

(22) Anmeldetag: **16.08.80**

(54) **Neue Diisocyanate bzw. Diisocyanatgemische der Diphenylmethanreihe, Verfahren zu ihrer Herstellung, sowie ihre Verwendung als Aufbaukomponente bei der Herstellung von Polyurethankunststoffen nach dem Isocyanat-Polyadditionsverfahren.**

(30) Priorität: **31.08.79 DE 2935318**

(43) Veröffentlichungstag der Anmeldung: **11.03.81 Patentblatt 81/10**

(45) Bekanntmachung des Hinweises auf die Patenterteilung: **05.05.82 Patentblatt 82/18**

(84) Benannte Vertragsstaaten: **AT BE CH DE FR GB IT LI NL SE**

(56) Entgegenhaltungen:
**DE-A-1 901 993**
**DE-A1-2 631 168**
**DE-B-1 192 188**
**US-A-3 375 264**

(73) Patentinhaber: **BAYER AG, Zentralbereich Patente, Marken und Lizenzen, D-5090 Leverkusen 1, Bayerwerk (DE)**

(72) Erfinder: **Knöfel, Hartmut, Dr., Dülmener Weg 21, D-5068 Odenthal (DE)**
Erfinder: **Brockelt, Michael, Neuenhauser Weg 1, D-5060 Bergisch-Gladbach 2 (DE)**
Erfinder: **Wegener, Gerhard, Dr., Futterstrasse 33, D-5600 Wuppertal 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 665 B1

# Neue Diisocyanate bzw. Diisocyanatgemische der Diphenylmethanreihe, Verfahren zu ihrer Herstellung, sowie ihre Verwendung als Aufbaukomponente bei der Herstellung von Polyurethankunststoffen nach dem Isocyanat-Polyadditionsverfahren

Die Erfindung betrifft neue, gegebenenfalls Methyl-substituierte Diisocyanate der Diphenylmethanreihe, die insbesondere durch Isocyanatgruppen in 3,4'- und gegebenenfalls in 3,2'-Stellung gekennzeichnet sind, mehrere, voneinander unabhängige Verfahren zu ihrer Herstellung, sowie ihre Verwendung als Isocyanatkomponente bei der Herstellung von Polyurethankunststoffen nach dem Isocyanat-Polyadditionsverfahren.

Unter den technisch und wirtschaftlich als Ausgangsmaterial für Polyurethankunststoffe wichtigen organischen Polyisocyanaten sind insbesondere 2,4-Diisocyanatotoluol bzw. dessen Gemische mit 2,6-Diisocyanatotoluol (TDI) und 4,4'-Diisocyanatodiphenylmethan bzw. dessen Gemische mit 2,2'- oder 2,4'-Diisocyanatodiphenylmethan und/oder mit höherfunktionellen Homologen (MDI) von hervorragender Bedeutung. Obwohl diese aromatischen Polyisocyanate in grosstechnischen Mengen weltweit zur Herstellung von Polyurethankunststoffen, insbesondere von Schaumstoffen und Elastomeren Verwendung finden, sind sie mit bestimmten Nachteilen behaftet. So weist beispielsweise TDI einen im Vergleich zu MDI erhöhten Dampfdruck auf, was bei der Verarbeitung dieses Rohstoffs aus physiologischen Gründen die strikte Beachtung entsprechender Vorsichtsmassnahmen erfordert. Andererseits ist MDI, d.h. insbesondere das auch in Polyisocyanatgemischen der Diphenylmethanreihe meistens als Hauptkomponente vorliegende 4,4'-Diisocyanatdiphenylmethan bei Raumtemperatur ein Festkörper mit einer hohen Neigung zur Kristallisation, so dass diese Rohstoffe oft vor ihrer Verarbeitung entweder durch Erwärmen auf eine Temperatur oberhalb des Schmelzpunkts von 4,4'-Diisocyanatodiphenylmethan oder durch eine chemische Modifizierung beispielsweise eine partielle Urethanisierung (vgl. z.B. US-PS 3 644 457) oder eine partielle Carbodiimidisierung (vgl. z.B. US-PS 3 152 162) verflüssigt werden müssen.

Durch die vorliegende Erfindung werden nunmehr neue aromatische Polyisocyanate zur Verfügung gestellt, die die Vorteile, nicht jedoch die Nachteile von TDI und MDI in sich vereinen. Darüber hinaus weisen die neuen Diisocyanate bzw. Diisocyanatgemische im Unterschied zu 4,4'-Diisocyanatodiphenylmethan Isocyanatgruppen unterschiedlicher Reaktivität auf, was oft bei der Herstellung von Polyurethankunststoffen von Vorteil ist. Ausserdem gestattet der Einsatz der neuen erfindungsgemässen Diisocyanate bzw. Diisocyanatgemische bei der Herstellung von Polyurethankunststoffen den Einbau neuartiger, dem Kohlenwasserstoffgerüst der Diisocyanate entsprechender Hartsegmente in die Polyurethankunststoffe, was eine interessante neue Variationsmöglichkeit bei der Herstellung von Polyurethankunststoffen eröffnet.

Gegenstand der vorliegenden Erfindung sind Diisocyanate der Formel

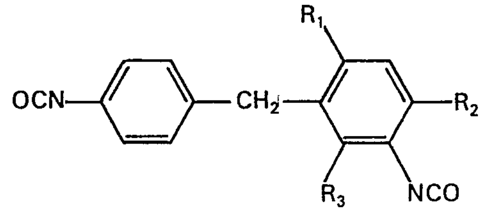

gegebenenfalls im Gemisch mit bis zu 40 Gew.-% bezogen auf Gesamtgemisch, an Diisocyanaten der Formel

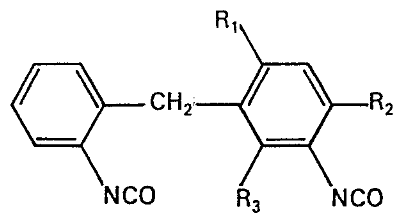

und gegebenenfalls mit bis zu 30 Gew.-%, bezogen auf Gesamtgemisch, an anderen, gegebenenfalls Methyl-substituierten Diisocyanatodiphenylmethan-Isomeren, wobei in diesen Formeln die Reste $R_1$, $R_2$ und $R_3$ jeweils gleich oder verschieden sind und Wasserstoff oder eine Methylgruppe bedeuten, mit der Einschränkung, dass in jeder der beiden Formeln jeweils mindestens 2 der Reste $R_1$, $R_2$ und $R_3$ für Wasserstoff stehen, und wobei vorzugsweise einer der Reste $R_1$, $R_2$ oder $R_3$ für eine Methylgruppe steht.

Die hier vorgenommene Definition für $R_1$, $R_2$ und $R_3$ gilt auch nachstehend.

Gegenstand der vorliegenden Erfindung ist auch das in diesen Diisocyanaten bzw. Diisocyanatgemischen meistens als Hauptkomponente vorliegende und auch in reiner Form darstellbare Diisocyanat der Formel

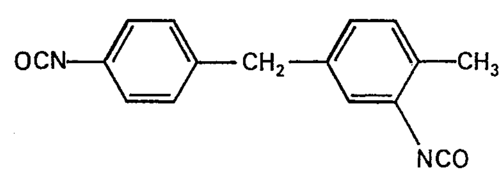

sowie das ebenfalls erfindungsgemässe Diisocyanat der Formel

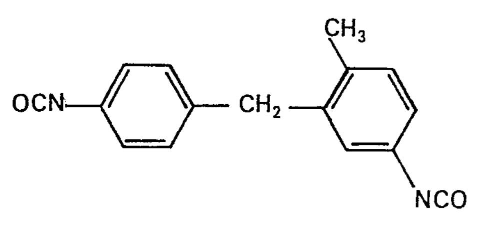

Gegenstand der vorliegenden Erfindung sind

auch die nachstehend näher beschriebenen Verfahren zur Herstellung der neuen Diisocyanate bzw. Diisocyanatgemische, sowie die Verwendung der neuen Diisocyanatgemische als Isocyanatkomponente bei der Herstellung von Polyurethankunststoffen nach dem Isocyanat-Polyadditionsverfahren.

Die oben und nachstehend gemachten Ausführungen bezüglich der Zusammensetzungen der erfindungsgemässen Gemische, sowie der Ausgangsmaterialien und Zwischenprodukte beziehen sich auf gaschromatographisch ermittelbare Werte.

Das 1. erfindungsgemässe Verfahren zur Herstellung von erfindungsgemäsen Diisocyanaten ist dadurch gekennzeichnet, dass man

a) ein 4-Nitrobenzylhalogenid in Gegenwart von Friedel-Crafts-Katalysatoren mit Nitrobenzol und/oder o-Nitrotoluol und/oder p-Nitrotoluol zu Dinitroverbindungen der Formel

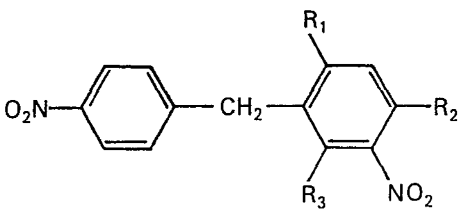

umsetzt, das Umsetzungsprodukt von eingesetztem Katalysator befreit,

b) das gemäss a) erhaltene Umsetzungsprodukt durch Hydrierung oder Reduktion der Nitrogruppen in die entsprechenden aromatischen Diaminoverbindungen überführt und

c) die gemäss b) erhaltenen Diaminoverbindungen durch Phosgenierung in die Diisocyanate überführt,

wobei man gegebenenfalls

d) aus den gemäss b) erhaltenen Diaminen vor der Phosgenierungsreaktion und/oder aus den gemäss c) erhaltenen Diisocyanaten die entsprechenden 3,4'-Diamino- bzw. 3,4'-Diisocyanato-Isomeren destillativ in von Nebenprodukten befreiter Form isoliert.

Unter «Nitrobenzylhalogenid» bzw. «Benzylhalogenid» sind hier und nachstehend vor allem die entsprechenden Benzylchloride bzw. -bromide, insbesondere die entsprechenden Benzylchloride zu verstehen.

Bei der Stufe a) des 1. erfindungsgemässen Verfahrens erfolgt eine Friedel-Crafts-Kondensation zwischen 4-Nitrobenzylhalogenid mit Nitrobenzol und/oder o-Nitrotoluol und/oder p-Nitrotoluol, wobei die Reaktionspartner in solchen Mengen zum Einsatz gelangen, dass für jedes Mol Nitrobenzylhalogenid 1,0 bis 20, vorzugsweise 2 bis 10 Mol Nitrobenzol und/oder Nitrotoluol zur Verfügung stehen. Die im Überschuss eingesetzte Komponente dient dabei gleichzeitig als Lösungsmittel. Als Katalysatoren dienen die üblichen Friedel-Crafts-Katalysatoren, d. h. z. B. Aluminiumchlorid, Eisentrichlorid, Titantetrachlorid oder Zinntetrachlorid. Vorzugsweise wird Eisentrichlorid als Katalysator verwendet. Die Katalysatoren kommen im allgemeinen in Mengen von 1 bis 100, vorzugsweise 5 bis 50 Mol-%, bezogen auf die Benzylhalogenidkomponente zum Einsatz. Die Umsetzung wird im allgemeinen bei einer zwischen Raumtemperatur und dem Siedepunkt des Reaktionsgemisches, d.h. bei ca. +20 bis ca. 200°C, vorzugsweise bei 30 bis 120°C liegenden Temperatur durchgeführt. Im Anschluss an die Kondensationsreaktion wird der Katalysator vorzugsweise durch Auswaschen z. B. mit Wasser und gegebenenfalls verdünnter Salzsäure entfernt und das überschüssige, nicht umgesetzte Ausgangsmaterial abdestilliert.

Anschliessend erfolgt gemäss der Reaktionsstufe b) die Reduktion der Nitrogruppen in die entsprechenden aromatisch gebundenen Aminogruppen. Vorzugsweise erfolgt diese Reduktion durch katalytische Hydrierung beispielsweise unter Verwendung von Raney-Nickel oder Palladium als Katalysator. Die Hydrierung erfolgt im allgemeinen in alkoholischer Lösung, wobei als Lösungsmittel beispielsweise Methanol, Ethanol oder Isopropanol zum Einsatz gelangt. Die zu hydrierenden Nitroverbindungen werden hierbei im allgemeinen in Form einer 10–50 gew.-%igen Lösung eingesetzt. Die Hydrierung erfolgt, gegebenenfalls unter Druck, bei einer Temperatur von 20 bis 150°C, vorzugsweise 30 bis 100°C. Die Überführung der Nitrogruppen in die entsprechenden Aminogruppen kann selbstverständlich auch nach der an sich bekannten Reduktionsmethode unter Verwendung von beispielsweise Eisen, Zink oder Zinn als Reduktionsmittel erfolgen. Im Anschluss an die Überführung der Nitrogruppen in die entsprechenden Aminogruppen wird der Katalysator beispielsweise durch Filtration entfernt und das Lösungsmittel abdestilliert. Das als Rückstand anfallende Amin kann dann ohne weitere Aufarbeitung dem Reaktionsschritt c) zugeführt werden. Falls auf die Herstellung besonders reiner Verfahrensprodukte Wert gelegt wird, kann aus dem gemäss Reaktionsschritt b) anfallenden Amingemisch das den erfindungsgemässen Verfahrensprodukten konstitutionell entsprechende Diamin bzw. Diamingemisch auch durch Destillation in von Nebenprodukten befreiter Form hergestellt werden, bevor es dem Reaktionsschritt c) zugeführt wird. Unter «Nebenprodukten» sind hier auch nachstehend nicht identifizierte niedriger und/oder höher als die erfindungsgemässen Produkte bzw. Zwischenprodukte siedenden Bestandteile zu verstehen.

Die gegebenenfalls destillativ aufgearbeiteten Diamine werden anschliessend gemäss Reaktionsschritt c) in an sich bekannter Weise durch Phosgenierung in die entsprechenden Diisocyanate überführt. Als Lösungsmittel dient hierbei beispielsweise Chlorbenzol oder Dichlorbenzol. Die erfindungsgemässen Verfahrensprodukte

liegen schliesslich nach Abdestillieren des Hilfslösungsmittels als Rückstand vor und können gewünschtenfalls durch Destillation in von gegebenenfalls noch vorliegenden Nebenprodukten befreiter Form erhalten werden.

Das 1. erfindungsgemässe Verfahren gestattet bei Verwendung von Nitrobenzol die Herstellung von 3,4'-Diisocyanatodiphenylmethan, bei der bevorzugten Verwendung von Nitrotoluol als Ausgangsmaterial entstehen beim 1. erfindungsgemässen Verfahren im Falle des o-Nitrotoluols Isomerengemische, die ca. 30 bis 50 Gew.-% 3,4'-Diisocyanato-2-methyl-diphenylmethan und ca. 50 bis 70 Gew.-% 3,4'-Diisocyanato-4-methyl-diphenylmethan enthalten und im Falle des p-Nitrotoluols 3,4'-Diisocyanato-6-methyl-diphenylmethan. Eine Reindarstellung von 3,4'-Diisocyanato-4-methyl-diphenylmethan ist nach dem Prinzip des 1. erfindungsgemässen Verfahrens beispielsweise durch Reindarstellung des 3,4'-Dinitro-4-methyl-diphenylmethans durch partielle Kristallisation aus dem gemäss a) anfallenden Zwischenprodukt und seine weitere Umsetzung gemäss b) und c) möglich. Diese Reindarstellung durch Kristallisation auf der Nitrostufe erfolgt beispielsweise dergestalt, dass man das nach Abdestillieren von überschüssigem Ausgangsmaterial erhaltene Gemisch von Nitroverbindungen auf Basis von 4-Nitrobenzylhalogenid und o-Nitrotoluol in siedendem Alkohol oder Essigsäureethylester zu einer gesättigten Lösung löst und die Lösung auf Raumtemperatur abkühlen lässt. Hierbei kristallisiert bevorzugt das gewünschte Isomere aus. Der Kristallisationsvorgang kann selbstverständlich beliebig oft wiederholt werden.

Das 2. erfindungsgemässe Verfahren zur Herstellung erfindungsgemässer Diisocyanate bzw. Diisocyanatgemische ist dadurch gekennzeichnet, dass man

a) ein 4-Nitrobenzylhalogenid in Gegenwart von Friedel-Crafts-Katalysatoren mit Toluol zu einem Gemisch von Mononitroverbindungen der Formel

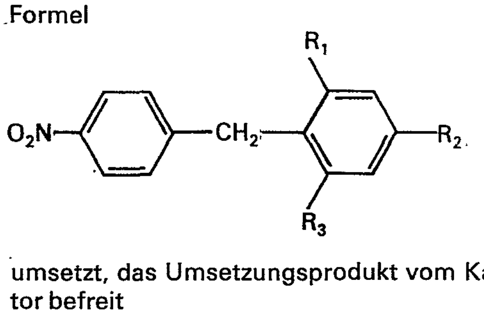

umsetzt, das Umsetzungsprodukt vom Katalysator befreit

b) das gemäss a) erhaltene Umsetzungsprodukt einer Nitrierungsreaktion unter Bildung von Dinitroverbindungen der Formel

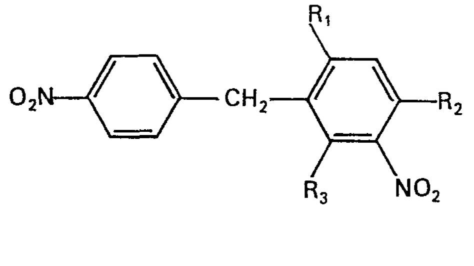

unterzieht,

c) die gemäss b) erhaltenen Dinitroverbindungen durch Hydrierung bzw. Reduktion der Aminogruppen in die entsprechenden aromatischen Diaminoverbindungen überführt und

d) die gemäss c) erhaltenen Diaminoverbindungen durch Phosgenierung in die entsprechenden Diisocyanate überführt,

wobei man gegebenenfalls

e) aus den gemäss c) erhaltenen Diaminen vor der Phosgenierungsreaktion und/oder aus den gemäss d) erhaltenen Diisocyanaten die entsprechenden 3,4'-Diamino- bzw. 3,4'-Diisocyanato-Isomeren destillativ in von Nebenprodukten befreiter Form isoliert.

Die Stufe a) des 2. erfindungsgemässen Verfahrens entspricht mit der Ausnahme der Verwendung von Toluol als Ausgangsmaterial der Stufe a) des 1. erfindungsgemäsen Verfahrens, wobei jedoch im allgemeinen die Siedetemperatur des hier in einem entsprechenden Überschuss eingesetzten Toluols die obere Grenze des Temperaturbereichs darstellt. Das gemäss a) erhaltene, vom Katalysator befreite Kondensationsprodukt wird anschliessend in der Stufe b) in an sich bekannter Weise zu einem Gemisch isomerer Dinitroverbindungen der genannten allgemeinen Formel nitriert. Diese Nitrierung erfolgt vorzugsweise in Gegenwart eines geeigneten Lösungsmittels wie beispielsweise Methylenchlorid unter Verwendung von «Nitriersäure», d. h. ein Gemisch aus konzentrierter Schwefelsäure und Salpetersäure, vorzugsweise hochkonzentrierter, ca. 98%iger Salpetersäure. Die Menge der Nitriersäure wird so bemessen, dass für jedes Mol an gemäss a) erhaltener Mononitroverbindung ca. 1,1 Mol Salpetersäure zur Verfügung stehen. Die Nitrierung wird im allgemeinen im Temperaturbereich zwischen −20 und 80°C, vorzugsweise 0-20°C, durchgeführt. Anschliessend wird die nach der Nitrierungsreaktion vorliegende organische Phase von der Säure durch Phasentrennung, Auswaschen mit Wasser und beispielsweise Natriumcarbonatlösung befreit. Schliesslich erfolgt die destillative Entfernung des Hilfslösungsmittels gegebenenfalls unter anschliessendem Austreiben von Lösungsmittelresten mittels Wasserdampf.

Die weitere Umsetzung der so erhaltenen Dinitroverbindungen erfolgt in völliger Analogie zu den Reaktionsschritten b) und c) des 1. erfindungsgemässen Verfahrens.

Beim 2. erfindungsgemässen Verfahren entstehen als erfindungsgemässe Diisocyanatgemische im allgemeinen Gemische, die ca. 70 bis 90 Gew.-% 3,4'-Diisocyanato-2-, -4- oder -6-methyldiphenylmethan und 10 bis 30 Gew.-% an anderen, analytisch nicht charakterisierten Methyl-substituierten Diisocyanato-diphenylmethan-Isomeren enthalten. Die Diisocyanate entsprechen somit in erster Näherung der Formel

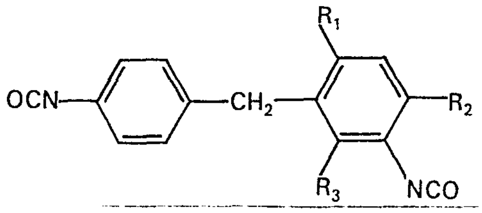

wobei — dies gilt auch für die beiden oben zuletzt genannten Formeln — einer der Reste $R_1$, $R_2$ oder $R_3$ für eine Methylgruppe und die beiden anderen der genannten Reste für Wasserstoff stehen.

Das 3. erfindungsgemässe Verfahren zur Herstellung erfindungsgemässer Diisocyanate bzw. Diisocyanatgemische ist dadurch gekennzeichnet, dass man

a) ein 3-Nitrobenzylhalogenid in Gegenwart von Friedel-Crafts-Katalysatoren mit Benzol zu 3-Nitro-diphenylmethan umsetzt, den Katalysator aus dem Umsetzungsprodukt entfernt,

b) die gemäss a) erhaltene Nitroverbindung einer Nitrierungsreaktion unter Bildung von bis zu 40 Gew.-% 2', 3-Dinitro-diphenylmethan enthaltendem 3,4'-Dinitrodiphenylmethan unterzieht,

c) die gemäss b) erhaltenen Dinitroverbindungen durch Hydrierung bzw. Reduktion der Nitrogruppen in die entsprechenden aromatischen Diaminoverbindungen überführt,

d) die gemäss c) erhaltenen Diaminoverbindungen durch Phosgenierung in die entsprechenden Diisocyanate überführt,

wobei man gegebenenfalls

e) aus den gemäss c) anfallenden Aminoverbindungen vor ihrer Phosgenierung oder aus den gemäss d) erhaltenen Polyisocyanaten destillativ das reine 3,4'-Isomere bzw. dessen Gemische mit bis zu 40 Gew.-% an 2', 3-Isomerem in reiner Form isoliert.

Abgesehen von der Verwendung anderer Ausgangsmaterialien entspricht das dritte erfindungsgemässe Verfahren völlig dem bereits geschilderten 2. Verfahren. Als Verfahrensprodukt entsteht im wesentlichen 3,4'-Diisocyanatdiphenylmethan, welches bis zu 40 Gew.-%, im allgemeinen 20 bis 30 Gew.-%, bezogen auf Gesamtgemisch, an 2,3'-Diisocyanatodiphenylmethan enthält.

Das 4. erfindungsgemässe Verfahren ist dadurch gekennzeichnet, dass man

a) technische, durch Nitrierung von Benzylchlorid erhaltene, 10 bis 50 Gew.-%, bezogen auf Gesamtgemisch, 2-Nitrobenzylchlorid und 50 bis 90 Gew.-%, bezogen auf Gesamtgemisch, 4-Nitrobenzylchlorid neben untergeordneten Mengen an 3-Nitrobenzylchlorid enthaltende Nitrobenzylchlorid-Isomerengemische in Gegenwart von Friedel-Crafts-Katalysatoren mit Toluol zu einem im wesentlichen Mononitroverbindungen der Formeln

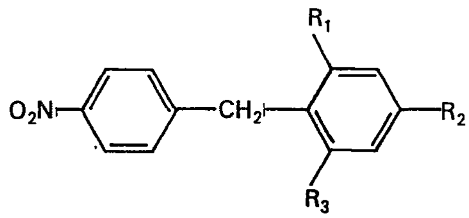

und

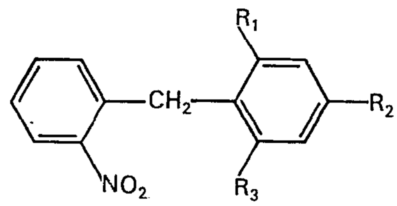

enthaltenden Friedel-Crafts-Kondensat umsetzt, den Katalysator aus dem Kondensat entfernt,

b) das gemäss a) erhaltene Kondensat einer Nitrierungsreaktion unter Bildung von im wesentlichen Nitroverbindungen der Formeln

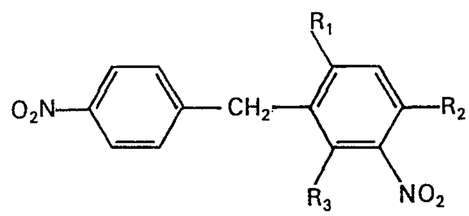

und

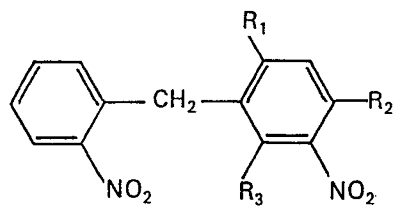

enthaltenden Gemisch aromatischer Dinitroverbindungen unterzieht,

c) die gemäss b) erhaltenen Nitroverbindungen durch Hydrierung bzw. Reduktion der Nitrogruppen in die entsprechenden aromatischen Diamine überführt,

d) die gemäss c) erhaltenen Diamine durch Phosgenierung in die entsprechenden Diisocyanate überführt,

wobei man gegebenenfalls

e) aus dem gemäss c) anfallenden Gemisch an aromatischen Aminoverbindungen von der Phosgenierung und/oder aus dem gemäss d) erhaltenen Polyisocyanatgemisch destillativ das 30 bis 60 Gew.-%, bezogen auf Gesamtgemisch, an 3,4'-Isomeren und 10 bis 40 Gew.-%, bezogen auf Gesamtgemisch an 3,2'-Isomeren sowie 0–30 Gew.-%, bezogen auf Gesamtgemisch, an anderen methylsubstituierten Diisocyanatodiphenylmethan-Isomeren enthaltende Isomerengemisch in von Nebenprodukten befreiter Form isoliert.

Die Durchführung des 4. erfindungsgemässen Verfahrens erfolgt in völliger Analogie zum 2. erfindungsgemässen Verfahren, wobei man jedoch anstelle von 4-Nitrobenzylhalogenid als Ausgangsmaterial ein technisches Nitrobenzylchlorid-Isomerengemisch verwendet, welches 10 bis 50 Gew.-%, vorzugsweise 30 bis 40 Gew.-% 2-Nitrobenzylchlorid und 50 bis 90 Gew.-% vorzugsweise 50 bis 60 Gew.-% 4-Nitrobenzylchlorid neben untergeordneten Mengen an 3-Nitrobenzylchlorid enthält. Unter «untergeordneten Mengen» sind hierbei, bezogen auf Gesamtgewicht maximal 20 Gew.-%, vorzugsweise maximal 15 Gew.-% zu verstehen. 3-Nitrobenzylchlorid liegt im Ausgangsgemisch stets in einer geringeren, d. h. untergeordneten Menge als die beiden anderen Isomeren vor.

Beim 4. erfindungsgemässen Verfahren entstehen als Verfahrensprodukte erfindungsgemässe Diisocyanatgemische, welche 30 bis 60 Gew.-%, vorzugsweise 40 bis 55 Gew.-%, bezogen auf Gesamtgemisch aus Diisocyanaten der Formel

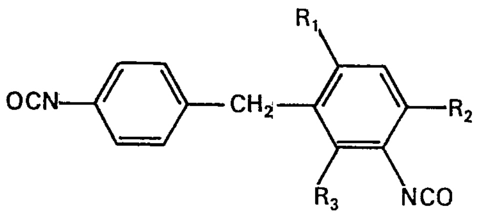

und 10 bis 40 Gew.-%, vorzugsweise 20 bis 35 Gew.-% bezogen auf Gesamtgemisch, an Diisocyanaten der Formel

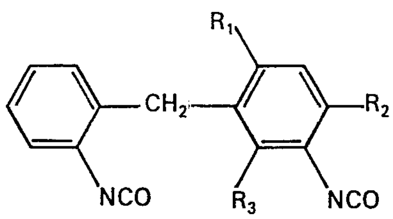

sowie bis zu 30 Gew.-%, bezogen auf Gesamtgemisch, an anderen analytisch nicht charakterisierbaren Methyl-substituierten Diisocyanatodiphenylmethan-Isomeren enthalten, wobei in den beiden Formeln ebenso wie in den vier zuletzt genannten Formeln für die Nitroverbindungen, jeweils einer der Reste $R_1$, $R_2$ oder $R_3$ für eine Methylgruppe und die beiden anderen Reste jeweils für Wasserstoff stehen.

Das 5. erfindungsgemässe Verfahren ist dadurch gekennzeichnet, dass man

a) technische, durch Nitrierung von 4-Methylbenzylchlorid erhaltene, 10 bis 25 Gew.-%, bezogen auf Gesamtgemisch, 2-Nitro-4-methyl-benzylchlorid und 75 bis 90 Gew.-%, bezogen auf Gesamtgemisch, 3-Nitro-4-methyl-benzylchlorid enthaltende Nitrobenzylchlorid-Isomerengemische in Gegenwart von Friedel-Crafts-Katalysatoren mit Benzol zu einem im wesentlichen Mononitroverbindungen der Formeln

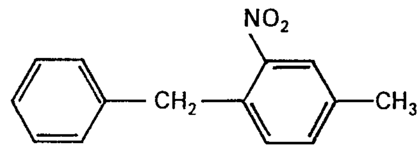

und als Hauptkomponente

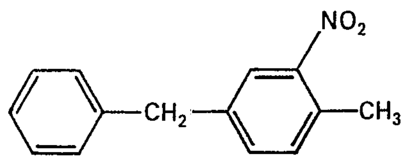

enthaltenden Friedel-Crafts-Kondensat umsetzt, den Katalysator aus dem Kondensat entfernt,

b) das gemäss a) erhaltene Kondensat einer Nitrierungsreaktion unter Bildung von im wesentlichen Nitroverbindungen der Formeln

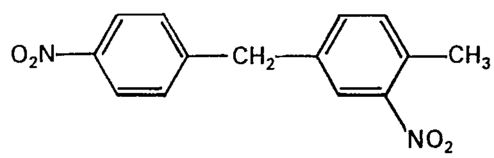

und

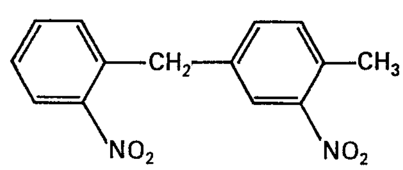

enthaltenden Gemisch aromatischer Dinitroverbindungen unterzieht,

c) die gemäss b) erhaltene Nitroverbindungen durch Hydrierung bzw. Reduktion der Nitrogruppen in die entsprechenden aromatischen Diamine überführt,

d) die gemäss c) erhaltenen Diamine durch Phosgenierung in die entsprechenden Diisocyanate überführt,

wobei man gegebenenfalls

e) aus dem gemäss c) anfallenden Gemisch an aromatischen Aminoverbindungen vor der Phosgenierung und/oder aus dem gemäss d) erhaltenen Polyisocyanatgemisch destillativ das 30 bis 60 Gew.-%, bezogen auf Gesamtgemisch, an 3,4'-Isomeren und 10 bis 40 Gew.-%, bezogen auf Gesamtgemisch an 3,2'-Isomeren sowie 0–30 Gew.-%, bezogen auf Gesamtgemisch, an anderen Methyl-substituierten Diisocyanatodiphenylmethan-Isomeren enthaltende Isomerengemisch in von Nebenprodukten befreiter Form isoliert.

Die Durchführung des 5. erfindungsgemässen Verfahrens erfolgt in völliger Analogie zum 4. erfindungsgemässen Verfahren, wobei man jedoch anstelle von technischen Nitrobenzylhalogenid als Ausgangsmaterial ein technisches 4-Methylnitrobenzylchlorid-Isomerengemisch verwendet, welches 10 bis 25 Gew.-%, vorzugsweise 15 bis 22 Gew.-%, 4-Methyl-2-nitrobenzylchlorid und 75 bis 90 Gew.-%, vorzugsweise 78 bis 85 Gew.-% 4-Methyl-3-nitrobenzylchlorid enthält.

Beim 5. erfindungsgemässen Verfahren entstehen als Verfahrensprodukte erfindungsgemässe Diisocyanatgemische, welche 30 bis 60 Gew.-%, vorzugsweise 40 bis 55 Gew.-%, bezogen auf Gesamtgemisch aus Diisocyanaten der Formel

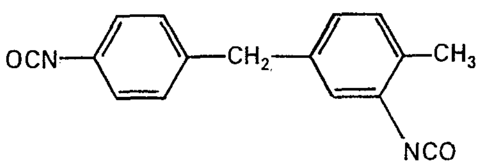

und 10 bis 40 Gew.-%, vorzugsweise 20 bis 30 Gew.-%, bezogen auf Gesamtgemisch der Formel

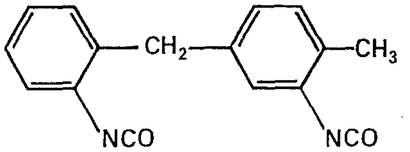

sowie bis zu 30 Gew.-%, bezogen auf Gesamtgemisch an anderen analytisch nicht charakterisierbaren Methyl-substituierten Diisocyanatodiphenylmethan-Isomeren enthalten.

Das 6. erfindungsgemässe Verfahren zur Herstellung von erfindungsgemässen Diisocyanaten bzw. Diisocyanatgemischen ist dadurch gekennzeichnet, dass man

a) ein Benzylhalogenid in Gegenwart von Friedel-Crafts-Katalysatoren oder Benzylalkohol in Gegenwart von Säure-Katalysatoren mit Toluol zu einem im wesentlichen aus Kohlenwasserstoffen der Formel

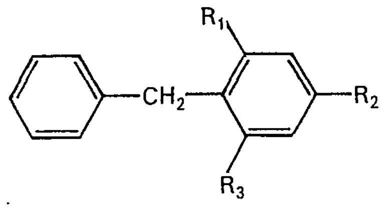

bestehenden Kondensat umsetzt,

b) das aus dem gemäss a) erhaltenen Kondensat destillativ in reiner Form erhaltene Methyl-diphenylmethan-Isomerengemisch der zuletzt genannten Formel einer Dinitrierung unterzieht.

c) die gemäss b) erhaltenen Dinitroverbindungen durch Hydrierung bzw. Reduktion der Nitrogruppen in die entsprechenden Diamine überführt,

d) die gemäss c) erhaltenen Diamine durch Phosgenierung in die entsprechenden Diisocyanate überführt,

wobei man gegebenenfalls

e) aus den gemäss c) erhaltenen Diaminen vor der Phosgenierung und/oder aus den gemäss d) erhaltenen Diisocyanatgemischen das entsprechende im Gemisch mit bis zu 40 Gew.-% 2',3-Isomeren und mit bis zu 30 Gew.-% bezogen auf Gesamtgemisch, an anderen Methyl-substituierten Diisocyanatodiphenylmethan-Isomeren vorliegende 3,4'-Isomere in von Nebenprodukten befreiter Form isoliert.

Bei der Stufe a) des 6. erfindungsgemässen Verfahrens wird anstelle eines Nitrobenzylhalogenids das unsubstituierte Benzylhalogenid oder Benzylalkohol eingesetzt.

Im Falle der Verwendung eines Benzylhalogenids gelten bezüglich der Durchführung der Stufe a) insbesondere bezüglich der Mengenverhältnisse der Reaktionspartner die bei der Schilderung des 1. und 2. erfindungsgemässen Verfahrens gemachten Ausführungen. Vorzugsweise wird jedoch bei einem Molverhältnis von Toluol zu Benzylchlorid von 5:1 bis 15:1 gearbeitet, besonders bevorzugt ist 8:1 bis 12:1. Die Kondensationsreaktion kann jedoch im Extremfall in der Gasphase bei Temperaturen von bis zu 300°C durchgeführt werden. Die bevorzugte Temperatur zur Durchführung der Stufe a) liegt jedoch innerhalb der für die Stufe a) des 2. erfindungsgemässen Verfahrens gültigen Bereiche.

Im Falle der Verwendung von Benzylalkohol als Ausgangsmaterial werden als Katalysatoren schwerflüchtige starke Säuren eingesetzt wie z. B. Schwefelsäure, Phosphorsäure oder beispielsweise Sulfonsäuregruppen aufweisende Festbettkatalysatoren wie Sulfonsäuregruppen aufweisende Ionenaustauscher oder anorganische saure Zentren aufweisende Feststoffkatalysatoren (Tonsile, Zeolithe etc).

Bezüglich der Mengenverhältnisse der Reaktionspartner gelten die für die Reaktion zwischen Benzylchlorid und Toluol gemachten Ausführungen sinngemäss, d. h. auch hier wird das Toluol in einem diesen Ausführungen entsprechenden Überschuss eingesetzt. Die Reaktionstemperatur liegt hier im allgemeinen zwischen −20 und 300, vorzugsweise 20 bis 110°C. Das anfallende Kondensat wird beispielsweise durch Auswaschen mit Wasser bei homogener Katalyse, bzw. durch Filtrieren bei heterogener Katalyse vom Katalysator und durch Abdestillieren vom überschüssigen Toluol befreit und in destillativ von geringen Mengen an höhermolekularen Kondensaten befreiter Form der Stufe b) zugeführt.

Bei dieser Reaktionsstufe b) wird das Kondensat einer Dinitrierung unterzogen, wobei im Prinzip die bei der Schilderung der Stufe b) des 2. erfindungsgemässen Verfahrens gemachten Ausführungen gelten, jedoch mit dem Unterschied, dass hier für jedes Mol an zu nitrisierendem Kohlenwasserstoff eine solche Menge an Nitriersäure zur Verfügung steht, die ca. 2,0 bis 2,5 Mol Salpetersäure entspricht.

Die weitere Verarbeitung der so erhaltenen Methyl-substituierten Dinitrodiphenylmethan-Isomeren erfolgt in völliger Analogie zu der bereits in Zusammenhang mit dem 1. oder 2. erfindungsgemässen Verfahren gemachten Ausführungen. Auch hier kann, wie beim 1. erfindungsgemässen Verfahren beschrieben, durch partielle Kristallisation auf der Nitro-Stufe das 4-Methyl-3,4'-Isomere dargestellt werden.

Beim 6. erfindungsgemässen Verfahren werden im Gemisch mit bis zu 40 Gew.-%, vorzugsweise im Gemisch mit 10 bis 25 Gew.-%, bezogen auf Gesamtgemisch an Diisocyanaten der Formel

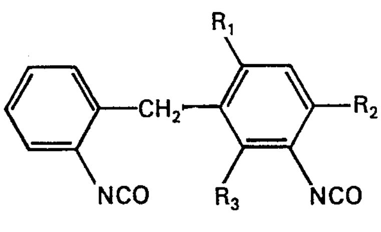

und mit bis zu 30 Gew.-%, bezogen auf Gesamtgemisch an anderen Methyl-substituierten Diisocyanatodiphenylmethan-Isomeren vorliegende Diisocyanate der Formel

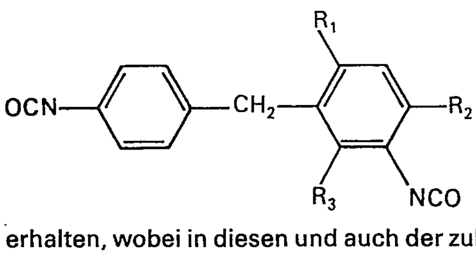

erhalten, wobei in diesen und auch der zuletzt genannten Formel für die durch die Kondensationsreaktion entstehenden Kohlenwasserstoffe jeweils einer der Reste $R_1$, $R_2$ oder $R_3$ für eine Methylgruppe und die beiden anderen Reste jeweils für Wasserstoff stehen.

Das 7. erfindungsgemässe Verfahren zur Herstellung erfindungsgemässer Diisocyanate bzw. Diisocyanatgemische ist dadurch gekenzeichnet, dass man

a) ein p-Methylbenzylhalogenid in Gegenwart von Friedel-Crafts-Katalysatoren mit Benzol zu 4-Methyl-diphenylmethan und höhermolekularen Kondensaten bestehenden Kohlenwasserstoffgemisch umsetzt,

b) das aus dem gemäss a) erhaltene Kohlenwasserstoffgemisch destillativ in reiner Form erhaltene 4-Methyl-diphenylmethan dinitriert,

c) die gemäss b) erhaltene Dinitroverbindung durch Hydrierung oder Reduktion der Nitrogruppen in die entsprechende aromatische Diaminoverbindung überführt,

d) die gemäss c) erhaltenen Diamine durch Phosgenierung in die entsprechenden Diisocyanate überführt,

wobei man gegebenenfalls

e) aus den gemäss c) erhaltenen Diaminen vor der Phosgenierungsreaktion und/oder aus den gemäss d) erhaltenen Diisocyanaten die entsprechenden im Gemisch mit bis zu 30 Gew.-%, bezogen auf Gesamtgemisch, an 2',3-Isomeren und bis zu 30 Gew.-%, bezogen auf Gesamtgemisch, an anderen Methyl-substituierten Diisocyanatodiphenylmethan-Isomeren vorliegenden 3,4'-Isomeren in von Nebenprodukten befreiter Form isoliert.

Bezüglich der einzelnen Verfahrensschritte gelten hier die bei der Schilderung des 6. erfindungsgemässen Verfahrens gemachten Ausführungen in vollem Umfang.

Beim 7. erfindungsgemässen Verfahren wird im Gemisch mit bis zu 30 Gew.-%, vorzugsweise 10 bis 20 Gew.-%, bezogen auf Gesamtgemisch, an 2',3-Diisocyanato-4-methyl-diphenylmethan sowie mit bis zu 30 Gew.-%, vorzugsweise bis 20 Gew.-%, bezogen auf Gesamtgemisch, an anderen analytisch nicht charakterisierbaren Methyl-substituierten Diisocyanatodiphenylmethan-Isomeren vorliegendes 3,4'-Diisocyanato-4-methyl-diphenylmethan erhalten.

Im Falle der Herstellung von Methyl-substituierten erfindungsgemässen Diisocyanaten bzw. Diisocyanatgemischen entstehen, vom 1. erfindungsgemässen Verfahren unter Verwendung von Nitrobenzol und p-Nitrotoluol und vom 3. und 7. erfindungsgemässen Verfahren abgesehen, stets Gemische von sich bezüglich der Stellung des Methylsubstituenten unterscheidenden Isomeren, wobei meistens das erfindungsgemässe 3,4'-Diisocyanato-4-methyl-diphenyl-methan den Hauptbestandteil bildet. Bezüglich der Eigenschaften der erfindungsgemässen Diisocyanatgemische und insbesondere bezüglich deren Eignung als Ausgangsmaterial zur Herstellung von Polyurethanen ist die Stellung des Methylsubstituenten bzw. der jeweilige Anteil der jeweiligen,

sich durch die Stellung des Methylsubstituenten unterscheidenden Isomeren von völlig untergeordneter Bedeutung. Die erfindungsgemässen Polyisocyanatgemische stellen technische Gemische dar, die, wie dargelegt, oftmals untergeordnete Mengen, d. h. bis zu 30 Gew.-% an analytisch, d. h. insbesondere gaschromatographisch nicht eindeutig charakterisierbaren Bestandteilen enthalten. Es handelt sich hierbei im allgemeinen um einen Methylsubstituenten aufweisende Diisocyanato-diphenylmethan-Isomere, deren NCO-Gruppen in 2,4′, 4,4′- oder 3,3′-Stellung angeordnet sind, bzw. um Gemische derartiger Isomerer. Der Gehalt der erfindungsgemässen Gemische an diesen Nebenprodukten in den angegebenen Grenzen beinträchtigt jedoch in keiner Weise deren vorteilhaften Eigenschaften. Allen erfindungsgemässen Diisocyanaten bzw. Diisocyanatgemischen gemeinsam sind die eingangs erwähnten Vorteile im Vergleich zu TDI bzw. MDI. Die erfindungsgemässen Diisocyanate bzw. Diisocyanatgemische weisen bei einem Druck von 0.1 mbar im allgemeinen einen Siedepunkt von ca. 125 bis 165°C auf. Die Viskosität der erfindungsgemässen Diisocyanate bzw. Diisocyanatgemische bei 25°C liegt im allgemeinen im Bereich von 10 bis 35 mPa.s. Die erfindungsgemässen Diisocyanatgemische weisen bei Raumtemperatur keinerlei Tendenz zur Kristallisation auf. Das erfindungsgemässe 4-Methyl-3,4′-Diisocyanatodiphenylmethan weist zwar einen Schmelzpunkt von ca. 42°C auf, zeigt beim Lagern bei Raumtemperatur im Unterschied zu 4,4′-Diisocyanatodiphenylmethan eine nur geringe Kristallisationsneigung.

Die erfindungsgemässen Diisocyanate bzw. Diisocyanatgemische insbesondere die erfindungsgemäss bevorzugten Methyl-substituierten Verbindungen bzw. deren Gemische stellen besonders wertvolle neuartige Ausgangsmaterialien zur Herstellung von Polyurethankunststoffen dar. Die neuen erfindungsgemässen Diisocyanate bzw. Diisocyanatgemische können bei allen bislang bekanntgewordenen Verfahren zur Polyurethankunststoffherstellung unter Verwendung von TDI und/oder MDI an Stelle dieser Polyisocyanate des Standes der Technik eingesetzt werden.

Beispiel 1

a) Bei Raumtemperatur wurden 171,6 g (1 Mol) 4-Nitrobenzylchlorid in 615 g (5 Mol) Nitrobenzol gelöst und mit 16,2 g (0,1 Mol) wasserfreiem Eisen-III-chlorid versetzt.

Unter Rühren wurde langsam aufgeheizt bis auf 100°C, 3 Stunden bei dieser Temperatur gehalten und beim Nachlassen der Chlorwasserstoffentwicklung noch 1 Stunde bei 120°C nachgerührt. Nach dem Abkühlen auf Raumtemperatur wurde mit je 200 ml verdünnter Salzsäure und dreimal Wasser gewaschen. Die getrocknete organische Phase wurde an der Ölpumpe vom überschüssigen Nitrobenzol befreit. Der Rückstand betrug 236 g.

b) 225 g des Rückstandes aus der Kondensationsstufe wurden in 500 ml Methanol aufgenommen, mit 50 g frischem Raney-Nickel versetzt und bei 50°C mit Wasserstoff bei 50 bar bis zur Druckkonstanz reduziert. Nach Beendigung der exothermen Reaktion wurde noch eine Stunde bei 80°C und 50 bar Wasserstoffdruck gehalten. Danach wurde entspannt, der Katalysator abfiltriert und Methanol und Wasser abdestilliert.

Der Rückstand (173 g) bestand nach gaschromatographischer Analyse aus

93 Gew.-% 3,4′-Diaminodiphenylmethan
7 Gew.-% Drei- (> 6 Gew.-%) und Höher- (< 1 Gew.-%) Kernverbindungen.

c) Das Rohamin (172 g) wurde in 1,25 l Monochlorbenzol gelöst und bei 0°C unter Rühren und Kühlen zu einer Lösung von 400 g Phosgen in 1,25 l Monochlorbenzol langsam zugetropft. Anschliessend wurde unter weiterem Durchleiten von Phosgen das Reaktionsgemisch langsam aufgeheizt und eine Stunde am Rückfluss gekocht.

Im Wasserstrahlvakuum wurde anschliessend das Monochlorbenzol abgezogen und bei weiter reduziertem Druck (0,05 Torr) das Isocyanat destilliert. Nach einem Vorlauf von ca. 5 ml gingen bei 130–2°C 184 g (0,74 Mol) 3,4′-Diisocyanatodiphenylmethan über. Als Destillationsrückstand verblieben 19,5 g.

Beispiel 2

a) Bei Raumtemperatur wurden 171,6 g (1 Mol) 4-Nitrobenzylchlorid in 685 g (5 Mol) 2-Nitrotoluol gelöst und mit 16,2 g (0,1 Mol) wasserfreiem Eisen-III-chlorid versetzt.

Unter Rühren wurde das Reaktionsgemisch langsam aufgeheizt auf 120°C und bis zum Nachlassen der Chlorwasserstoffentwicklung bei dieser Temperatur gehalten. Danach wurde noch eine Stunde bei 120°C nachgerührt.

Nach dem Abkühlen auf Raumtemperatur wurde mit 500 ml Methylenchlorid verdünnt, einmal mit 200 ml halbkonzentrierter Salzsäure extrahiert und dreimal mit je 500 ml Wasser gewaschen. Nach dem Trocknen wurde zunächst das Methylenchlorid und dann das überschüssige 2-Nitrotoluol abgetrennt, gefolgt von einer Wasserdampfdestillation.

Der Rückstand (230 g) enthielt nach gaschromatographischer Analyse:

2 Gew.-% Niedermolekulare Bestandteile
92 Gew.-% Zweikernisomere
6 Gew.-% Drei- und Mehrkernisomere

Die Zweikernfraktion bestand aus:

61,1 Gew.-% 4- } Methyl-3,4′-dinitrodi-
38,9 Gew.-% 2- } phenylmethan

b) Das Rohprodukt der Nitrostufe wurde insgesamt analog Beispiel 1b) durch Reduktion in die Aminstufe überführt.

c) Das Rohgemisch der Amine wurde analog Beispiel 1c) durch Phosgenieren in die Isocyanate übergeführt.

Im Wasserstrahlvakuum wurde anschliessend das Monochlorbenzol abgezogen und bei weiter reduziertem Druck (0,1 Torr) das Isocyanat destilliert. Nach einem geringen Vorlauf (ca. 5 ml) ging bei 155–165°C eine Diisocyanatfraktion (166 g; 0,63 Mol) über bei 19 g Destillationsrückstand.

Die Diisocyanatfraktion bestand nach GC aus:

39 Gew.-% 2- } Methyl-3,4'-diisocyanato-
61 Gew.-% 4- } diphenylmethan

Beispiel 3

a) 171,6 g (1 Mol) 4-Nitrobenzylchlorid wurden zusammen mit 685 g (5 Mol) p-Nitrotoluol geschmolzen und nach dem Homogenisieren mit 32,4 (0,2 Mol) wasserfreiem Eisen-III-chlorid versetzt.

Die Durchführung der Reaktion und die Aufarbeitung der Produkte erfolgte analog Beispiel 2a) und ergab einen festen Rückstand (224 g).

b) Das Rohprodukt der Nitrostufe wurde insgesamt analog Beispiel 1b) durch Reduktion in die Aminstufe übergeführt.

c) Das Rohgemisch der Amine wurde analog Beispiel 1c) durch Phosgenieren in die Isocyanate übergeführt.

Das Rohisocyanat (198 g) wurde bei reduziertem Druck destilliert. Die Hauptfraktion 171,5 g (0,65 Mol) bestand aus:
6-Methyl-3,4'-diisocyanatodiphenylmethan
($Kp_{0,05}$ 143°C, farblose Flüssigkeit).

Beispiel 4

a) 343,0 g (2 Mol) 4-Nitrobenzylchlorid wurden in 920 g (10 Mol) trockenem Toluol gelöst und mit 32,4 g (0,2 Mol) wasserfreiem Eisen-III-chlorid versetzt.

Unter Rühren wurde langsam bis auf 80°C aufgeheizt, 5 Stunden bei dieser Temperatur gehalten, nach beendeter Chlorwasserstoffentwicklung 1 Stunde am Rückfluss gekocht, nach dem Abkühlen in Eiswasser gegossen und dreimal mit je 500 ml Wasser säurefrei gewaschen.

Nach dem Trocknen und Abdestillieren des überschüssigen Toluols bei Normaldruck wurde der Rückstand (438 g) bei vermindertem Druck (0,5 Torr) destilliert. Zwischen 140 und 150°C gingen 398,4 g (87,8% d.Th.) eines niederviskosen gelben Öles über.

340,5 g (1,5 Mol) des destillierten Gemisches der 4-Nitromethyldiphenylmethane wurden in 40 ml Methylenchlorid bei 0–10°C vorgelegt. Unter Rühren und Kühlen wurden bei dieser Temperatur Nitriersäure zugegeben, bestehend aus einer Mischung von

110 g (1,7 Mol) 98 %iger $HNO_3$ und
170 g (1,7 Mol) 98 %iger $H_2SO_4$.

Nach beendeter Zugabe wurde noch 30 Minuten bei 10°C nachgerührt und dann die Mischsäure abgetrennt. Die verbleibende organische Phase wurde zweimal mit je 200 ml Wasser, einmal mit 5%iger Natriumcarbonatlösung und noch zweimal mit Wasser gewaschen. Anschliessend wurde das Methylenchlorid bei Normaldruck bis 90°C Sumpftemperatur abdestilliert, und die Lösungsmittelreste wurden mit Wasserdampf ausgetrieben. Das resultierende Produkt wurde im Wasserstrahlvakuum bei 80°C entwässert. Es enthielt nach GC:

2,4 Gew.-% Mono- }
91,7 Gew.-% Di- } nitromethyldiphenylmethane
5,9 Gew.-% Tri- }

c) Das Rohprodukt der Nitrostufe (397 g) wurde insgesamt analog Beispiel 1b) durch Reduktion in die Aminstufe übergeführt.

d) Das Rohgemisch der Amine (296 g) wurde analog Beispiel 1c) durch Phosgenierung in die Isocyanate übergeführt.

Im Wasserstrahlvakuum wurde anschliessend das Monochlorbenzol abgezogen und bei weiter reduziertem Druck (0,1 Torr) das Isocyanat destilliert. Nach einem Vorlauf von ca. 25 ml ging bei 155–165°C eine Diisocyanatfraktion (295,3 g; 1,12 Mol) über. Der Destillationsrückstand betrug 52 g.

Die Diisocyanatfraktion bestand nach GC aus:

ca. 80 Gew.-% 2-, 4- und 6-Methyl-3,4' diisocyanatodiphenylmethan und
ca. 20 Gew.-% anderen Isomeren.

Beispiel 5

a) 171,5 g (1 Mol) 3-Nitrobenzylchlorid wurden in 546 g (7 Mol) trockenem Benzol gelöst und mit 13 g (0,1 Mol) wasserfreiem Aluminiumchlorid versetzt.

Das Reaktionsgemisch wurde langsam erhitzt bis das Benzol am Rückfluss kochte. Die bei ca. 60°C einsetzende Chlorwasserstoffabspaltung war nach ca. 1 Stunde beendet und es wurde eine weitere Stunde unter Rückfluss nachgerührt.

Die Aufarbeitung erfolgte analog Beispiel 4a). Bei der Destillation des nach Abziehen des Benzols verbleibenden Rückstandes gingen bei 0,1–0,5 Torr zwischen 95 und 135°C 182 g (0,858 Mol) einer Fraktion über, die nach GC zu 96 Gew.-% aus meta-Nitrodiphenylmethan bestand. Der Destillationsrückstand betrug 21 g.

b) 213 g (1 Mol) 3-Nitro-diphenylmethan wurden einer Zweitnitrierung unterworfen analog Beispiel 4b) und aufgearbeitet. Das Rohprodukt (248 g) enthielt nach GC:

1,6 Gew.-% Mono }
96,2 Gew.-% Di- } nitrodiphenylmethan
2,2 Gew.-% Tri- }

c) Das Rohprodukt der Nitrostufe wurde insgesamt analog Beispiel 1b) durch Reduktion in die Aminstufe übergeführt (Ausbeute: 180,5 g).

d) Das Rohgemisch der Amine wurde analog Beispiele 1c) durch Phosgenieren in die Isocyanate übergeführt: (Ausbeute 230 g).

Im Wasserstrahlvakuum wurde anschliessend das Monochlorbenzol abgezogen und bei weiter reduziertem Druck (0,1 Torr) das Isocyanat destilliert. Nach einem Vorlauf von ca. 15 ml, der auch noch Chlorbenzol enthielt, ging bei 145–160°C eine Diisocyanatfraktion 199 g (0,8 Mol) über. Der Destillationsrückstand betrug 17 g.

Die Diisocyanatfraktion hatte folgende Zusammensetzung (GC):

22 Gew.-% 2,3'-<br>
5,2 Gew.-% 3,3'-<br>
72,8 Gew.-% 3,4'-<br>
} diisocyanato-diphenylmethan

Beispiel 6

a) 254 g (2 Mol) Benzylchlorid wurden in 400 ml Methylenchlorid zusammen mit 295 g (3 Mol) 98 %iger Schwefelsäure bei −5°C vorgelegt. Unter Rühren und Kühlen wurden dazu bei −5°C bis 0°C im Verlauf von 1,5 Stunden 190 g (3 Mol) 100 %iger Salpetersäure zugetropft. Nach beendeter Zugabe wurde noch eine Stunde bei 0°C nachgerührt.

Das Nitriergemisch wurde anschliessend in 1,2 l Eiswasser eingerührt, die organische Phase abgetrennt, zweimal mit je 500 ml Waser gewaschen, mit 300 ml gesättigter Natriumcarbonatlösung geschüttelt, noch einmal mit Wasser gewaschen und über Natriumsulfat getrocknet.

Bei Normaldruck wurde das Methylenchlorid abgezogen. Der Rückstand wurde bei vermindertem Druck (0,1 Torr) destilliert. Dabei gingen zwischen 80°C und 120°C Kopftemperatur 328 g eines Gemisches der Mononitrobenzylchloride über. Der Destillationsrückstand betrug 6 g, so dass bei weiteren Ansätzen auf eine Destillation verzichtet wurde.

Die Isomerenverteilung der Hauptfraktion wurde indirekt bestimmt durch Umsetzung des Gemisches mit Benzol gemäss Beispiel 4a), jedoch in Gegenwart äquimolarer Mengen an wasserfreiem Eisen-III-chlorid. In dem resultierenden Gemisch der Mononitrodiphenylmethane wurde folgende Zusammensetzung gefunden:

GC: 33,7 Gew.-% 2-<br>
11,6 Gew.-% 3-<br>
54,2 Gew.-% 4-<br>
} Nitrodiphenylmethan

920 (10 Mol) trockenes Toluol wurden zusammen 121,5 g (0,75 Mol) wasserfreiem Eisen-III-chlorid bei 60°C vorgelegt. Unter Rühren und Kühlen (Chlorwasserstoffentwicklung) wurde langsam eine Lösung von 257,4 g (1,5 Mol) des Gemisches der Nitrobenzylchloride in 460 g (5 Mol) trockenem Toluol zugetropft. Nach beendeter Zugabe wurde noch 1 Stunde bei 80°C nachgerührt.

Die weitere Aufarbeitung erfolgte analog Beispiel 4a), wobei bei einem Druck von 0,1–0,3 Torr zwischen 115 und 140°C (296 g 1,30 Mol) ein Gemisch der Mononitro-methyldiphenylmethane überging (Destillationsrückstand: 39 g), das gaschromatographisch nicht zuzuordnen war.

b) Das Destillat (295 g) von 6a) wurde einer Zweitnitrierung und Aufarbeitung unterworfen analog Beispiel 4b. Ausbeute 341 g.

c) Das Rohgemisch der Nitroverbindungen wurde insgesamt analog Beispiel 1b) durch Reduktion in die Aminstufe übergeführt. Ausbeute 251 g.

d) Das Rohgemisch der Amine wurde analog Beispiel 1c) durch Phosgenieren in die Isocyanate übergeführt.

Im Wasserstrahlvakuum wurde anschliessend das Monochlorbenzol abgezogen und bei weiter reduziertem Druck (0,1 Torr) das Isocyanat destilliert. Nach einem Verlauf von ca. 18 ml ging bei 155–165°C eine Diisocyanatfraktion (251 g; 0,95 Mol) über. Der Destillationsrückstand betrug 44 g.

Die Diisocyanatfraktion bestand nach GC aus:

ca. 30 Gew.-% Methyl-2',3-diisocyanatodiphenylmethanen<br>
ca. 45 Gew.-% Methyl-3,4'-diisocyanatodiphenylmethanen<br>
ca. 25 Gew.-% andere Isomere

und fiel an als hellgelbes, niederviskoses Öl, (20 cp) ohne Neigung zur Kristallisation (bis 0°C untersucht).

Der Gehalt an 4-Methyl-3,4'-diisocyanato-diphenylmethan betrug ca. 31 Gew.-% (Hauptbestandteil).

Beispiel 7

a) 282 g (2 Mol) 4-Methylbenzylchlorid wurden analog Beispiel 6a) nitriert und aufgearbeitet. Ausbeute 362 g Rohprodukt.

Das Rohprodukt wurde analog 6a) mit Benzol umgesetzt, aufgearbeitet und nach dem Abziehen des Benzols destilliert. Bei 0,1 Torr wurde zwischen 115° und 125°C ein Gemisch der Mononitromethyldiphenylmethane erhalten (372 g = 1,64 Mol), das sich nach GC zusammensetzte aus:

ca. 19 Gew.-% 2-Nitro<br>
ca. 81 Gew.-% 3-Nitro<br>
} -4-methyldiphenylmethan

b) Das destillierte Gemisch (340,5 g = 1,5 Mol) wurde analog Beispiel 4b) einer Zweitnitrierung unterworfen. Ausbeute 396 g.

Nach GC ergab sich folgende Zusammensetzung:

1 Gew.-% Mononitro<br>
92 Gew.-% Dinitro<br>
7 Gew.-% Trinitro<br>
} -4-methyldiphenylmethan

c) Das Rohgemisch der Nitroverbindung wurde insgesamt analog Beispiel 1b) durch Reduktion in die Aminstufe übergeführt. Ausbeute 300 g.

d) Das Rohgemisch der Amine wurde insgesamt analog Beispiel 1c) durch Phosgenierung in die Isocyanate übergeführt.

Im Wasserstrahlvakuum wurde anschliessend das Monochlorbenzol abgezogen und bei weiter reduziertem Druck (0,1 Torr) das Isocyanat destilliert. Nach einem Vorlauf von ca. 15 ml, der noch Chlorbenzol enthielt, ging bei 155–165°C eine Diisocyanatfraktion (294 g = 1,11 Mol) über. Der Destillationsrückstand betrug 53 g.

Die Diisocyanatfraktion bestand nach GC aus:

25,2 Gew.-% 2',3-Diisocyanato-4-methyl-diphenylmethan
48,8 Gew.-% 3,4'-Diisocyanato-4-methyl-diphenylmethan
26 Gew.-% andere Isomere

und fiel an als hellgelbes, niederviskoses Öl.

Beispiel 8

a) Unter Stickstoff wurden 1,84 kg (20 Mol) trockenes Toluol vorgelegt und mit 2 g wasserfreiem Eisen-III-chlorid versetzt. Unter Rühren und Kochen am Rückfluss wurden anschliessend 253 g (2 Mol) trockenes Benzylchlorid zugetropft, wobei gasförmiger Chlorwasserstoff entwich.

Nach beendeter Zugabe wurde noch 30 Minuten nachgerührt, abgekühlt und dreimal mit je 500 ml Wasser säurefrei gewaschen. Anschliessend wurde aus der organischen Phase durch Destillation bei Normaldruck bis zu einer Sumpftemperatur von 150°C das überschüssige Toluol abgetrennt.

Das verbleibende Kohlenwasserstoffgemisch (346 g) bestand aus

89–92 Gew.-% Zweikernisomeren
8–10 Gew.-% Dreikernisomeren
<2 Gew.-% höherkernigen Bestandteilen.

Durch Destillation bei vermindertem Druck wurde zunächst die Zweikernfraktion ($kp_{0,1}$ 78–80°C) und anschliessend die Dreikernfraktion ($kp_{0,1}$ 150–175°C) abgetrennt.

Die Zweikernfraktion enthielt nach GC- und NMR-Analyse

44–45 Gew.-% 2-Methyldiphenylmethan
48–49 Gew.-% 4-Methyldiphenylmethan
6–8 Gew.-% 3-Methyldiphenylmethan

b) 364 g (2 Mol) des nach 8a) erhaltenen Gemisches der Zweikernisomeren des Methyldiphenylmethans wurden in 800 ml Methylenchlorid bei 0–10°C vorgelegt. Unter Rühren und Kühlen wurde bei dieser Temperatur Nitriersäure zugegeben, bestehend aus einer Mischung von

290 g (4,5 Mol) 98%iger $HNO_3$ und
450 g (4,5 Mol) 98%iger $H_2SO_4$

Bei 10°C wurde nach beendeter Zugabe noch 30 Minuten nachgerührt und dann die Mischsäure abgetrennt. Die organische Phase wurde zweimal mit je 400 ml Wasser, einmal mit 5%iger Natriumcarbonatlösung und noch zweimal mit Wasser gewaschen. Anschliessend wurde das Methylenchlorid bei Normaldruck bis 90°C Sumpftemperatur abdestilliert und die Lösungsmittelreste mit Wasserdampf im Wasserstrahlvakuum bei 80°C entwässert. Der Rückstand enthielt:

| | | |
|---|---|---|
| 5 Gew.-% | Nitrotoluol | |
| 2 Gew.-% | Mononitro | -methyl-diphenylmethan |
| 90 Gew.-% | Dinitro | |
| 2 Gew.-% | Tri- und Tetranitro | |

Die Dinitrofraktion besteht aus

15–20 Gew.-% 2',3- } Dinitromethyl-
55–60 Gew.-% 3,4'- } diphenylmethanen
bis zu 30 Gew.-% anderen Isomeren

c) 500 g des Rohgemisches der Nitrierstufe wurde in 1000 ml Methanol aufgenommen, mit 100 g frischem Raney-Nickel versetzt und bei 50°C mit einem Wasserstoffdruck von 50 bar reduziert. Nach Beendigung der exothermen Reaktion wurde bei 60°C noch 1 Stunde lang der Wasserstoffdruck auf 50 bar gehalten. Danach wurde entspannt, der Katalysator abfiltriert und Methanol und Wasser wurden abdestilliert.

Der Rückstand (381 g) wurde bei reduziertem Druck destilliert, wobei zunächst ein Vorlauf von ca. 10 g Toluidingemisch mit Wasserresten überging.

Bei 150–200°C (0,1 Torr) destillierte die Hauptmenge des Amingemisches (352 g). Der Rückstand im Destillationskolben ergab 19 g.

d) 212 g (1 Mol) des Gemisches der Diaminomethyldiphenylmethane (enthält noch ca. 1% Monoamin) wurden in 1,25 l Monochlorbenzol gelöst und bei 0°C unter Rühren und Kühlen zu einer Lösung von 400 g Phosgen in 1,25 l Monochlorbenzol langsam zugetropft. Anschliessend wurde unter weiterem Durchleiten von Phosgen das Reaktionsgemisch langsam aufgeheizt bis das Monochlorbenzol am Rückfluss kochte und so eine Stunde gehalten.

Nach dem Abziehen des Monochlorbenzols im Wasserstrahlvakuum wurde bei einem reduziertem Druck von 0,05 Torr das Isocyanat destilliert. Nach einem Vorlauf von 5 g erhielt man bei 155–165°C das Isomerengemisch der Isocyanate (248 g, NCO-Gehalt 31,7 g) als hellgelbes Öl, das selbst bei 0°C noch flüssig blieb. Der Destillationsrückstand ergab 11 g.

Die Isomerenverteilung der Diisocyanatfraktion entspricht der Isomerenverteilung der Dinitrofraktion.

Beispiel 9

a) 1000 g des gemäss Beispiel 8b) erhaltenen Rohgemisches der Nitroverbindungen wurden bei Raumtemperatur mit 1 l Ethanol zu einem Kri-

stallbrei verrührt und auf einer Nutsche abgesaugt. Der Rückstand wurde anschliessend einmal aus Isopropanol umkristallisiert. Der Rückstand des Kristallisationsschrittes (250 g = 0,92 Mol) bestand aus 3,4'-Dinitro-4-methyldiphenylmethan in ca. 98%iger Reinheit (Fp 146–147°C).

b) Die Dinitroverbindung wurde analog Beispiel 7c) zur Aminverbindung reduziert. Die Ausbeute an destilliertem Diamin betrug 180 g ($Kp_{0,1}$ 195°C) (0,85 Mol ≙ 92,4%).

c) Das Diamin (145 g = 0,68 Mol) wurde analog Beispiel 7d) in das Isocyanat übergeführt. Die Ausbeute nach der Destillation ($Kp_{0,1}$ 159–160°C) betrug 159 g (0,60 Mol) an

3,4'-Diisocyanato-4-methyldiphenylmethan.

Die Reinheit betrug nach GC 98,3%. Die farblose, ölige Flüssigkeit (Viskosität bei 25°C = 16 $C_p$) kristallisierte erst nach längerem Stehen ($F_p$ 41–42°C). Der NCO-Gehalt betrug 31,7 Gew.-%.

Beispiel 10

a) Unter Stickstoff wurden 5,85 kg (75 Mol) trockenes Benzol vorgelegt und mit 1 g waserfreiem Eisen-III-chlorid versetzt. Dazu wurden bei 60°C im Verlaufe von 2 Stunden eine Lösung von 591 g (4,2 Mol) 4-Methylbenzylchlorid in 702 g (9 Mol) Benzol zugetropft.

Nach beendeter Chlorwasserstoffentwicklung wurde noch 1 h am Rückfluss gekocht, abgekühlt, mit dreimal je 1 l Wasser säurefrei gewaschen und aus der organischen Phase bei Normaldruck das Benzol abdestilliert.

Das verbleibende Kohlenwasserstoffgemisch (ca. 700 g) wurde bei reduziertem Druck (0,1 Torr) destilliert. Bei 80°C gingen 294 g (38,5% d.Th.) 4-Methyldiphenylmethan über. Der Rest bestand aus 3- und höherkernigem Material, der bis auf einen geringen Rückstand zwischen 100 und 300°C destillierte.

b) 273 g (1,5 Mol) 4-Methyldiphenylmethan wurden analog Beispiel 8b) mit der entsprechenden Menge Nitriersäure in ein Gemisch von Nitroverbindungen übergeführt. Das resultierende Produkt (392 g) enthielt nach GC:

| | | | |
|---|---|---|---|
| 8 | Gew.-% | Nitroverb. des Toluols | |
| 1 | Gew.-% | Mononitro | 4-methyl-diphenyl-methan |
| 86,1 | Gew.-% | Dinitro | |
| 5 | Gew.-% | Trinitro | |

c) Das Rohgemisch der Nitrierstufe (290 g) wurde analog Beispiel 1b) bzw. 8c) durch Reduktion in die Aminstufte übergeführt. Das Amingemisch wurde im Wasserstrahlvakuum bis 200°C andestilliert.

Ausbeute 195 g Rohamin.

d) Das Rohgemisch der Amine wurde insgesamt analog Beispiel 1c) bzw. 8d) in die Isocyanate übergeführt.

Nach dem Abziehen des Monochlorbenzols im Wasserstrahlvakuum wurde bei 0,1 Torr das Isocyanat destilliert. Nach einem Vorlauf von 15 ml, der noch Monochlorbenzol und schon Produkt enthielt, ging bei 155–170°C das Isomerengemisch der Diisocyanate (274 g, NCO-Gehalt 31,7%) als hellgelbes Öl über, das selbst bei 0°C keine Neigung zur Kristallisation zeigte. Der Destillationsrückstand betrug 43 g.

Nach GC bestand das Gemisch aus

| | | | |
|---|---|---|---|
| 15,0 | Gew.-% | 2',3- | Diisocyanato-4-diphenyl methanen |
| 69,3 | Gew.-% | 3,4'- | |
| ca. 15 | Gew.-% | anderen | |

**Patentansprüche für die Vertragsstaaten: BE, CH, LI, DE, FR, GB, IT, NL, SE**

1. Diisocyanate der Formel

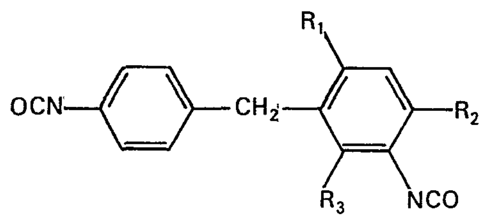

gegebenenfalls im Gemisch mit bis zu 40 Gew.-%, bezogen auf Gesamtgemisch, an Diisocyanaten der Formel

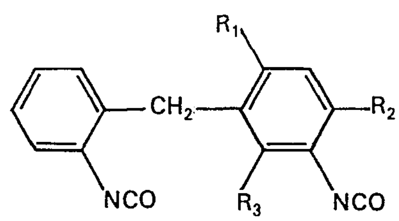

und gegebenenfalls mit bis zu 30 Gew.-%, bezogen auf Gesamtgemisch, an anderen, gegebenenfalls Methyl-substituierten Diisocyanatodiphenylmethan-Isomeren, wobei in diesen Formeln die Reste $R_1$, $R_2$ und $R_3$ jeweils gleich oder verschieden sind und Wasserstoff oder eine Methylgruppe bedeuten, mit der Einschränkung, dass in jeder der beiden Formeln jeweils mindestens 2 der Reste $R_1$, $R_2$ und $R_3$ für Wasserstoff stehen.

2. Diisocyanate bzw. Diisocyanatgemische gemäss Anspruch 1, dadurch gekennzeichnet, dass in beiden der in Anspruch 1 genannten Formeln jeweils einer der Reste $R_1$, $R_2$ oder $R_3$ für eine Methylgruppe steht.

3. Diisocyanat gemäss Anspruch 1 der Formel,

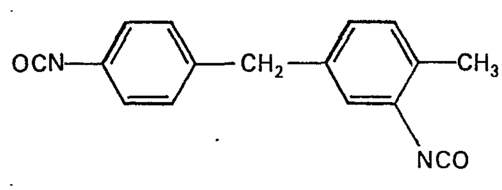

4. Diisocyanat gemäss Anspruch 1 der Formel

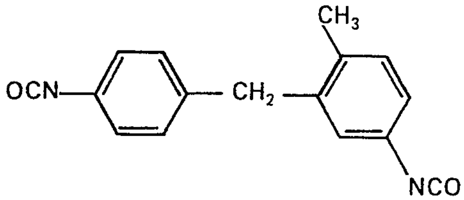

5. Verfahren zur Herstellung von Diisocyanaten der Formel

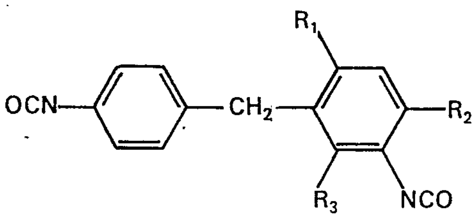

in welcher $R_1$, $R_2$ und $R_3$ die in Anspruch 1 genannte Bedeutung haben, dadurch gekennzeichnet, dass man

a) ein 4-Nitrobenzylhalogenid in Gegenwart von Friedel-Crafts-Katalysatoren mit Nitrobenzol und/oder o-Nitrotoluol und/oder p-Nitrotoluol zu Dinitroverbindungen der Formel

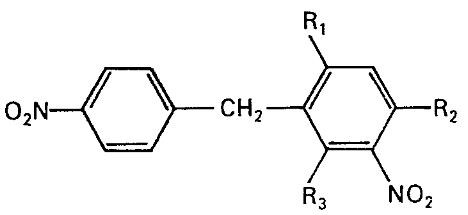

umsetzt, das Umsetzungsprodukt vom eingesetzten Katalysator befreit,

b) das gemäss a) erhaltene Umsetzungsprodukt durch Hydrierung oder Reduktion der Nitrogruppen in die entsprechenden aromatischen Diaminoverbindungen überführt und

c) die gemäss b) erhaltenen Diaminoverbindungen durch Phosgenierung in die Diisocyanate überführt,

wobei man gegebenenfalls

d) aus den gemäss b) erhaltenen Diaminen vor der Phosgenierungsreaktion und/oder aus den gemäss c) erhaltenen Diisocyanaten die entsprechenden 3,4'-Diamino- bzw. 3,4'-Diisocyanato-Isomeren destillativ in von Nebenprodukten befreiter Form isoliert.

6. Verfahren zur Herstellung von Diisocyanaten der Formel

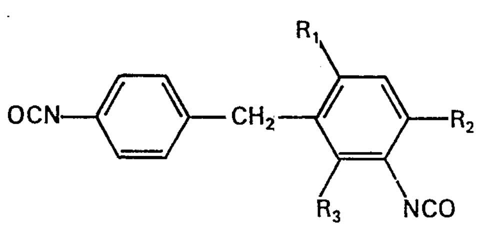

in welcher einer der Reste $R_1$, $R_2$ oder $R_3$ für eine Methylgruppe und die beiden anderen der genannten Reste für Wasserstoff stehen, dadurch gekennzeichnet, dass man

a) ein 4-Nitrobenzylhalogenid in Gegenwart von Friedel-Crafts-Katalysatoren mit Toluol zu einem Gemisch von Mononitroverbindungen der Formel

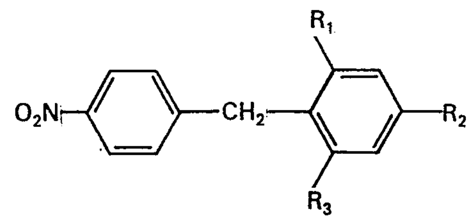

umsetzt, das Umsetzungsprodukt vom Katalysator befreit,

b) das gemäss a) erhaltene Umsetzungsprodukt einer Nitrierungsreaktion unter Bildung von Dinitroverbindungen der Formel

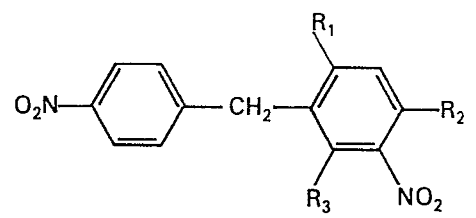

unterzieht,

c) die gemäss b) erhaltenen Dinitroverbindungen durch Hydrierung bzw. Reduktion der Aminogruppen in die entsprechenden aromatischen Diaminoverbindungen überführt und

d) die gemäss c) erhaltenen Diaminoverbindungen durch Phosgenierung in die entsprechenden Diisocyanate überführt,

wobei man gegebenenfalls

e) aus den gemäss c) erhaltenen Diaminen vor der Phosgenierungsreaktion und/oder aus den gemäss d) erhaltenen Diisocyanaten die entsprechenden 3,4'-Diamino- bzw. 3,4'-Diisocyanato-Isomeren destillativ in von Nebenprodukten befreiter Form isoliert.

7. Verfahren zur Herstellung von gegebenenfalls im Gemisch mit bis zu 40 Gew.-%, bezogen auf Gesamtgemisch an 2',3-Diisocyanato-diphenylmethan vorliegenden 3,4'-Diisocyanatodiphenylmethan, dadurch gekennzeichnet, dass man

a) ein 3-Nitrobenzylhalogenid in Gegenwart von Friedel-Crafts-Katalysatoren mit Benzol zu 3-Nitro-diphenylmethan umsetzt, den Katalysator aus dem Umsetzungsprodukt entfernt,

b) die gemäss a) erhaltene Nitroverbindung

einer Nitrierungsreaktion unter Bildung von bis zu 40 Gew.-% 2',3-Dinitro-diphenylmethan enthaltendem 3,4'-Dinitrodiphenylmethan unterzieht,

c) die gemäss b) enthaltenen Dinitroverbindungen durch Hydrierung bzw. Reduktion der Nitrogruppen in die entsprechenden aromatischen Diaminoverbindungen überführt,

d) die gemäss c) erhaltenen Diaminoverbindungen durch Phosgenierung in die entsprechenden Diisocyanate überführt,

wobei man gegebenenfalls

e) aus den gemäss c) anfallenden Aminoverbindungen vor ihrer Phosgenierung oder aus den gemäss d) erhaltenen Polyisocyanaten destillativ das reine 3,4'-Isomere bzw. dessen Gemische mit bis zu 40 Gew.-% an 3,2'-Isomerem in reiner Form isoliert.

8. Verfahren zur Herstellung von Diisocyanato-diphenylmethan-Isomerengemischen, welche 30 bis 60 Gew.-%, bezogen auf Gesamtgemisch an Diisocyanaten der Formel

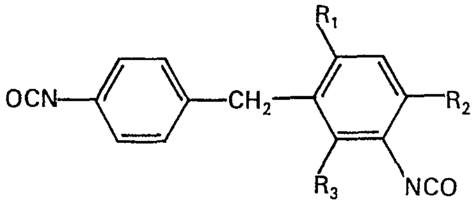

und 10 bis 40 Gew.-%, bezogen auf Gesamtgemisch, an Diisocyanaten der Formel

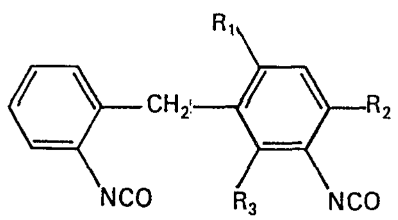

sowie 0 bis 30 Gew.-%, bezogen auf Gesamtgemisch, an anderen Methyl-substituierten Diisocyanatodiphenylmethan-Isomeren enthalten, wobei in beiden Formeln jeweils einer der Reste $R_1$, $R_2$ oder $R_3$ für eine Methylgruppe und die beiden anderen Reste jeweils für Wasserstoff stehen, dadurch gekennzeichnet, dass man

a) technische, durch Nitrierung von Benzylchlorid erhaltene, 10 bis 50 Gew.-%, bezogen auf Gesamtgemisch, 2-Nitrobenzylchlorid und 50 bis 90 Gew.-%, bezogen auf Gesamtgemisch, 4-Nitrobenzylchlorid neben untergeordneten Mengen an 3-Nitrobenzylchlorid enthaltende Nitrobenzylchlorid-Isomerengemische in Gegenwart von Friedel-Crafts-Katalysatoren mit Toluol zu einem im wesentlichen Mononitroverbindungen der Formeln

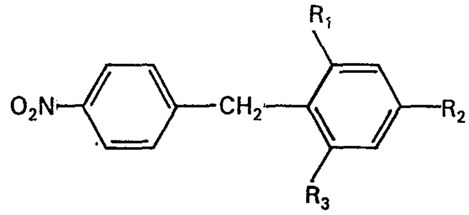

und

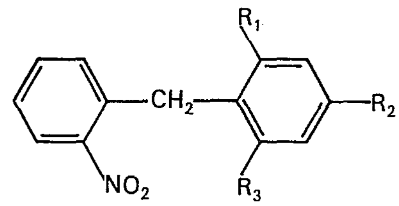

enthaltenen Friedel-Crafts-Kondensat umsetzt, den Katalysator aus dem Kondensat entfernt,

b) das gemäss a) erhaltene Kondesat einer Nitrierungsreaktion unter Bildung von im wesentlichen Nitroverbindungen der Formeln

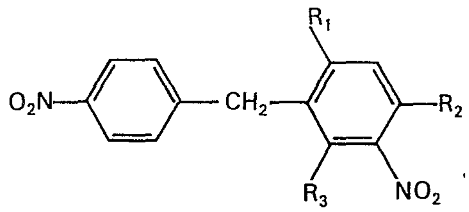

und

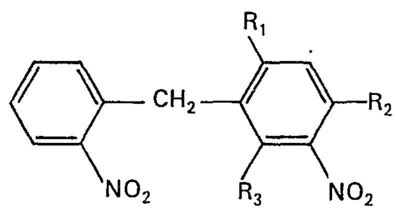

enthaltenden Gemisch aromatischer Dinitroverbindungen unterzieht,

c) die gemäss b) erhaltenen Nitroverbindungen durch Hydrierung bzw. Reduktion der Nitrogruppen in die entsprechenden aromatischen Diamine überführt.

d) die gemäss c) erhaltenen Diamine durch Phosgenierung in die entsprechenden Diisocyanate überführt,

wobei man gegebenenfalls

e) aus dem gemäss c) anfallenden Gemisch an aromatischen Aminoverbindungen vor der Phosgenierung und/oder aus dem gemäss d) erhaltenen Polyisocyanatgemisch destillativ das 30 bis 60 Gew.-%, bezogen auf Gesamtgemisch, an 3,4'-Isomeren und 10 bis 40 Gew.-%, bezogen auf Gesamtgemisch an 3,2'-Isomeren sowie 0–30 Gew.-%, bezogen auf Gesamtgemisch an anderen methylsubstituierten Diisocyanatodiphenylmethan-Isomeren enthaltende Isomerengemisch in von Nebenprodukten befreiter Form isoliert.

9. Verfahren zur Herstellung von Diisocyanato-diphenylmethan-Isomerengemischen, welche 30 bis 60 Gew.-%, bezogen auf Gesamtgemisch an Diisocyanaten der Formel

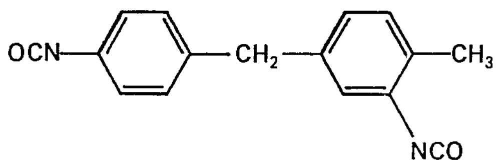

und 10 bis 40 Gew.-%, bezogen auf Gesamtgemisch, an Diisocyanaten der Formel

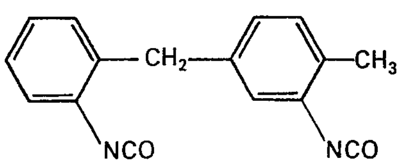

sowie 0 bis 30 Gew.-%, bezogen auf Gesamtgemisch, an anderen Methyl-substituierten Diisocyanatodiphenylmethan-Isomeren erhalten, wobei in beiden Formeln jeweils der Rest $R_2$ für eine Methylgruppe und die beiden anderen Reste jeweils für Wasserstoff stehen, dadurch gekennzeichnet, dass man

a) technische, durch Nitrierung von 4-Methylbenzylchlorid erhaltene, 10 bis 25 Gew.-%, bezogen auf Gesamtgemisch, 2-Nitro-4-methyl-benzylchlorid und 75 bis 90 Gew.-%, bezogen auf Gesamtgemisch, 3-Nitro-4-methyl-benzylchlorid enthaltende Nitrobenzylchlorid-Isomerengemische in Gegenwart von Friedel-Crafts-Katalysatoren mit Benzol zu einem im wesentlichen die Mononitroverbindungen der Formeln

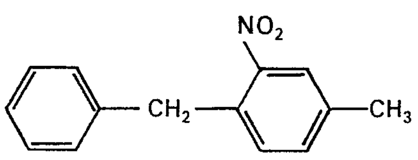

und als Hauptprodukt

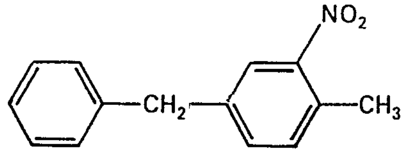

enthaltenden Friedel-Crafts-Kondensat umsetzt, den Katalysator aus dem Kondensat entfernt,

b) das gemäss a) erhaltene Kondensat einer Nitrierungsreaktion unter Bildung von im wesentlichen Nitroverbindungen der Formeln

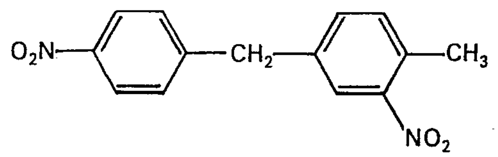

und

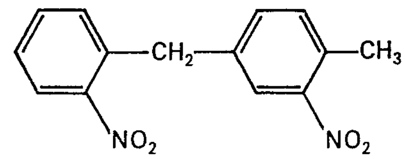

enthaltenden Gemisch aromatischer Dinitroverbindungen unterzieht,

c) die gemäss b) erhaltenen Nitroverbindungen durch Hydrierung bzw. Reduktion der Nitrogruppen in die entsprechenden aromatischen Diamine überführt,

d) die gemäss c) erhaltenen Diamine durch Phosgenierung in die entsprechenden Diisocyanate überführt,

wobei man gegebenenfalls

e) aus dem gemäss c) anfallenden Gemisch an aromatischen Aminoverbindungen vor der Phosgenierung und/oder aus dem gemäss d) erhaltenen Polyisocyanatgemisch destillativ das 30 bis 60 Gew.-%, bezogen auf Gesamtgemisch, an 3,4'-Isomeren und 10 bis 40 Gew.-%, bezogen auf Gesamtgemisch an 3,2'-Isomeren sowie 0–30 Gew.-%, bezogen auf Gesamtgemisch, an anderen methylsubstituierten Diisocyanatodiphenylmethan-Isomeren enthaltende Isomerengemisch in von Nebenprodukten befreiter Form isoliert.

10. Verfahren zur Herstellung von im Gemisch mit bis zu 40 Gew.-%, bezogen auf Gesamtgemisch, an Diisocyanaten der Formel

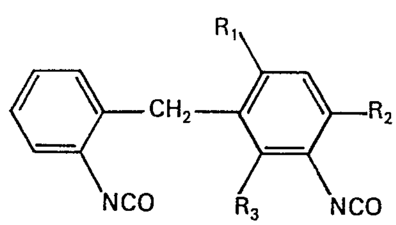

und mit bis zu 30 Gew.-%, bezogen auf Gesamtgemisch an anderen Methyl-substituierten Diisocyanatodiphenylmethan-Isomeren vorliegende Diisocyanate der Formel

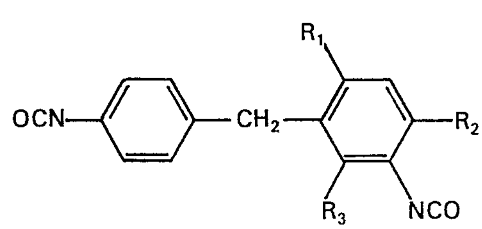

wobei in beiden Formeln jeweils einer der Reste $R_1$, $R_2$ oder $R_3$ für eine Methylgruppe und die beiden anderen Reste jeweils für Wasserstoff stehen, dadurch gekennzeichnet, dass man

a) ein Benzylhalogenid in Gegenwart von Friedel-Crafts-Katalysatoren oder Benzylalkohol in Gegenwart von Säure-Katalysatoren mit Toluol zu einem im wesentlichen aus Kohlenwasserstoffen der Formel

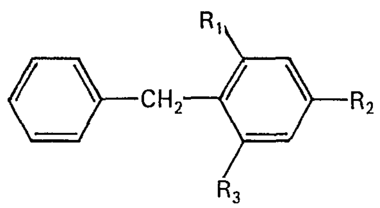

bestehenden Kondensat umsetzt,

b) das ausserdem gemäss a) erhaltenen Kondensat destillativ in reiner Form erhaltene Methyl-diphenylmethan-Isomerengemisch der zuletztgenannten Formel einer Dinitrierung unterzieht,

c) die gemäss b) erhaltenen Dinitroverbindungen durch Hydrierung bzw. Reduktion der Nitrogruppen in die entsprechenden Diamine überführt,

d) die gemäss c) erhaltenen Diamine durch Phosgenierung in die entsprechenden Diisocyanate überführt,

wobei man gegebenenfalls

e) aus den gemäss c) erhaltenen Diaminen vor der Phosgenierung und/oder aus den gemäss d) erhaltenen Diisocyanatgemischen das entsprechende im Gemisch mit bis zu 40 Gew.-% 2',3-Isomeren und mit bis zu 30 Gew.-%, bezogen auf Gesamtgemisch, an anderen Methyl-substituierten Diisocyanatodiphenylmethan-isomeren vorliegende 3,4'-Isomere in von Nebenprodukten befreiter Form isoliert.

11. Verfahren zur Herstellung von im Gemisch mit bis zu 40 Gew.-%, bezogen auf Gesamtgemisch, an 2',3-Diisocyanato-4-methyl-diphenylmethan und bis zu 30 Gew.-% bezogen auf Gesamtgemisch, an anderen Methyl-substituierten Diisocyanatodiphenylmethan-Isomeren vorliegenden 3,4'-Diisocyanato-4-methyl-diphenylmethan, dadurch gekennzeichnet, dass man

a) ein p-Methylbenzylhalogenid in Gegenwart von Friedel-Crafts-Katalysatoren mit Benzol zu einem aus 4-Methyl-diphenylmethan und höhermolekularen Kondensaten bestehenden Kohlenwasserstoffgemisch umsetzt,

b) das aus dem gemäss a) erhaltene Kohlenwasserstoffgemisch destillativ in reiner Form erhaltene 4-Methyl-diphenylmethan dinitriert,

c) die gemäss b) erhaltene Dinitroverbindung durch Hydrierung oder Reduktion der Nitrogruppen in die entsprechende aromatische Diamonoverbindung überführt,

d) die gemäss c) erhaltenen Diamine durch Phosgenierung in die entsprechenden Diisocyanate überführt,

wobei man gegebenenfalls

e) aus den gemäss c) erhaltenen Diaminen vor der Phosgenierungsreaktion und/oder aus den gemäss d) erhaltenen Diisocyanaten die entsprechenden im Gemisch mit bis zu 30 Gew.-%, bezogen auf Gesamtgemisch, an 2',3-Isomeren und bis zu 30 Gew.-%, bezogen auf Gesamtgemisch, an anderen Methyl-substituierten Diisocyanatodiphenylmethan-Isomeren vorliegenden 3,4'-Isomeren in von Nebenprodukten befreiter Form isoliert.

12. Verwendung der Diisocyanate bzw. Diisocyanatgemische gemäss Ansprüchen 1 bis 4 als Isocyanatkomponente bei der Herstellung von Polyurethankunststoffen nach dem Isocyanat-Polyadditionsverfahren.

**Patentansprüche für den Vertragsstaat: AT**

1. Verfahren zur Herstellung von Diisocyanaten der Formel

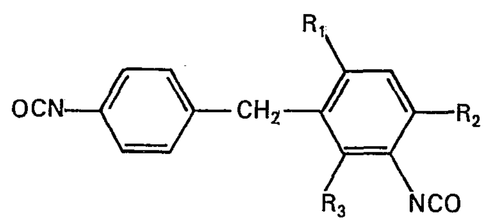

in welcher $R_1$, $R_2$ und $R_3$ jeweils gleich oder verschieden sind und Wasserstoff oder eine Methylgruppe bedeuten, mit der Einschränkung, dass in jeder der beiden Formeln jeweils mindestens zwei der Reste $R_1$, $R_2$ und $R_3$ für Wasserstoff stehen, dadurch gekenzeichnet, dass man

a) ein 4-Nitrobenzylhalogenid in Gegenwart von Friedel-Crafts-Katalysatoren mit Nitrobenzol und/oder o-Nitrotoluol und/oder p-Nitrotoluol zu Dinitroverbindungen der Formel

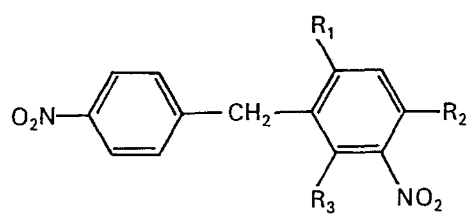

umsetzt, das Umsetzungsprodukt vom eingesetzten Katalysator befreit,

b) das gemäss a) erhaltene Umsetzungsprodukt durch Hydrierung oder Reduktion der Nitrogruppen in die entsprechenden aromatischen Diaminoverbindungen überführt und

c) die gemäss b) erhaltenen Diaminoverbindungen durch Phosgenierung in die Diisocyanate überführt,

wobei man gegebenenfalls

d) aus den gemäss b) erhaltenen Diaminen vor der Phosgenierungsreaktion und/oder aus den gemäss c) erhaltenen Diisocyanaten die entsprechenden 3,4′-Diamino- bzw. 3,4′-Diisocyanato-Isomeren destillativ in von Nebenprodukten befreiter Form isoliert.

2. Verfahren zur Herstellung von Diisocyanaten der Formel

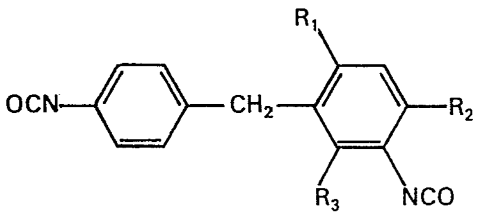

in welcher einer der Reste $R_1$, $R_2$ oder $R_3$ für eine Methylgruppe und die beiden anderen der genannten Reste für Wasserstoff stehen, dadurch gekennzeichnet, dass man

a) ein 4-Nitrobenzylhalogenid in Gegenwart von Friedel-Crafts-Katalysatoren mit Toluol zu einem Gemisch von Mononitroverbindungen der Formel

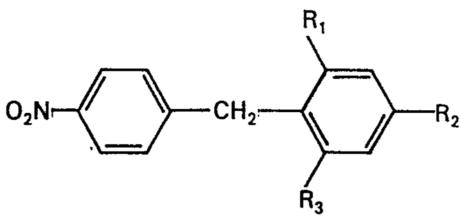

umsetzt, das Umsetzungsprodukt vom Katalysator befreit,

b) das gemäss a) erhaltene Umsetzungsprodukt einer Nitrierungsreaktion unter Bildung von Dinitroverbindungen der Formel

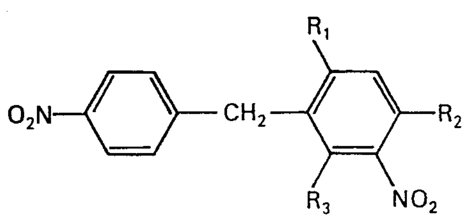

unterzieht,

c) die gemäss b) erhaltenen Dinitroverbindungen durch Hydrierung bzw. Reduktion der Aminogruppen in die entsprechenden aromatischen Diaminoverbindungen überführt und

d) die gemäss c) erhaltenen Diaminoverbindungen durch Phosgenierung in die entsprechenden Diisocyanate überführt,

wobei man gegebenenfalls

e) aus den gemäss c) erhaltenen Diaminen vor der Phosgenierungsreaktion und/oder aus den gemäss d) erhaltenen Diisocyanaten die entsprechenden 3,4′-Diamino- bzw. 3,4′-Diisocyanato-Isomeren destillativ in von Nebenprodukten befreiter Form isoliert.

3. Verfahren zur Herstellung von gegebenenfalls im Gemisch mit bis zu 40 Gew.-%, bezogen auf Gesamtgemisch an 2′,3-Diisocyanatodiphenylmethan vorliegenden 3,4′-Diisocyanatodiphenylmethan, dadurch gekennzeichnet, dass man

a) ein 3-Nitrobenzylhalogenid in Gegenwart von Friedel-Crafts-Katalysatoren mit Benzol zu 3-Nitro-diphenylmethan umsetzt, den Katalysator aus dem Umsetzungsprodukt entfernt,

b) die gemäss a) erhaltene Nitroverbindung einer Nitrierungsreaktion unter Bildung von bis zu 40 Gew.-% 2′,3-Dinitro-diphenylmethan enthaltendem 3,4′-Dinitrodiphenylmethan unterzieht,

c) die gemäss b) erhaltenen Dinitroverbindungen durch Hydrierung bzw. Reduktion der Nitrogruppen in die entsprechenden aromatischen Diaminoverbindungen überführt,

d) die gemäss c) erhaltenen Diaminoverbindungen durch Phosgenierung in die entsprechenden Diisocyanate überführt,

wobei man gegebenenfalls

e) aus den gemäss c) anfallenden Aminoverbindungen vor ihrer Phosgenierung oder aus den gemäss d) erhaltenen Polyisocyanaten destillativ das reine 3,4′-Isomere bzw. dessen Gemische mit bis zu 40 Gew.-% an 3,2′-Isomeren in reiner Form isoliert.

4. Verfahren zur Herstellung von Diisocyanatodiphenylmethan-Isomerengemischen, welche 30 bis 60 Gew.-%, bezogen auf Gesamtgemisch an Diisocyanaten der Formel

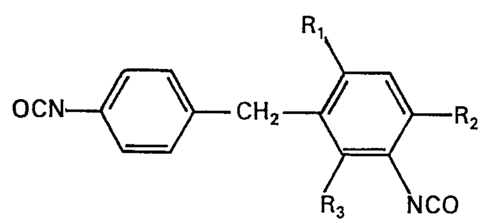

und 10 bis 40 Gew.-%, bezogen auf Gesamtgemisch, an Diisocyanaten der Formel

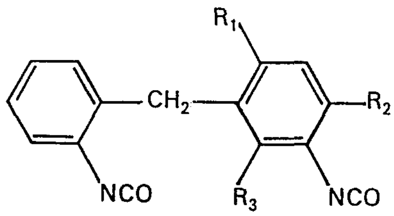

sowie 0 bis 30 Gew.-%, bezogen auf Gesamtgemisch, an anderen Methyl-substituierten Diisocyanatodiphenylmethan-Isomeren enthalten, wobei in beiden Formeln jeweils einer der Reste $R_1$, $R_2$ oder $R_3$ für eine Methylgruppe und die beiden anderen Reste jeweils für Wasserstoff stehen, dadurch gekennzeichnet, dass man

a) technische, durch Nitrierung von Benzylchlorid erhaltene, 10 bis 50 Gew.-%, bezogen auf Gesamtgemisch, 2-Nitrobenzylchlorid und 50 bis 90 Gew.-%, bezogen auf Gesamtgemisch, 4-Nitrobenzychlorid neben untergeordneten Mengen an 3-Nitrobenzylchlorid enthaltende Nitrobenzylchlorid-Isomerengemische in Gegenwart von Friedel-Crafts-Katalysatoren mit Toluol zu einem im wesentlichen Mononitroverbindungen der Formeln

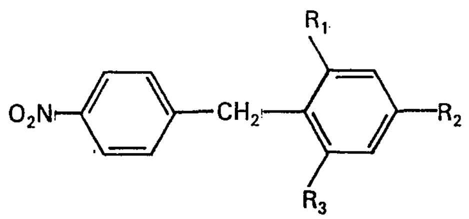

und

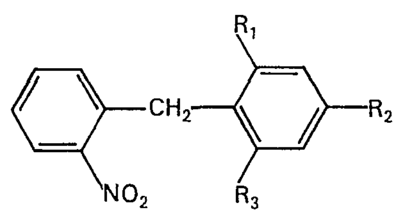

enthaltenen Friedel-Crafts-Kondensat umsetzt, den Katalysator aus dem Kondensat entfernt,b)

das gemäss a) erhaltene Kondensat einer Nitrierungsreaktion unter Bildung von im wesentlichen Nitroverbindungen der Formeln

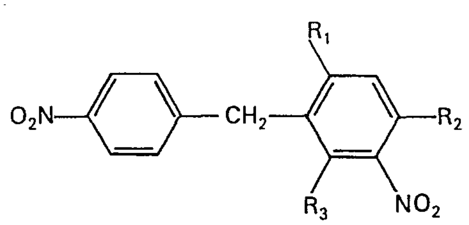

und

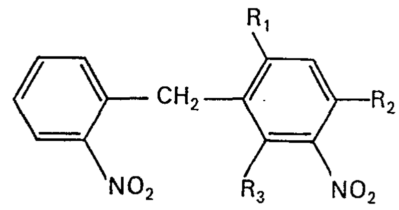

enthaltenden Gemisch aromatischer Dinitroverbindungen unterzieht,

c) die gemäss b) erhaltenen Nitroverbindungen durch Hydrierung bzw. Reduktion der Nitrogruppen in die entsprechenden aromatischen Diamine überführt,

d) die gemäss c) erhaltenen Diamine durch Phosgenierung in die entsprechenden Diisocyanate überführt,

wobei man gegebenenfalls

e) aus dem gemäss c) anfallenden Gemisch an aromatischen Aminoverbindungen vor der Phosgenierung und/oder aus dem gemäss d) erhaltenen Polyisocyanatgemisch destillativ das 30 bis 60 Gew.-%, bezogen auf Gesamtgemisch, an 3,4'-Isomeren und 10 bis 40 Gew.-%, bezogen auf Gesamtgemisch an 3,2'-Isomeren sowie 0–30 Gew.-%, bezogen auf Gesamtgemisch an anderen methylsubstituierten Diisocyanatodiphenylmethan-Isomeren enthaltende Isomerengemisch in von Nebenprodukten befreiter Form isoliert.

5. Verfahren zur Herstellung von Diisocyanatodiphenylmethan-Isomerengemisch, welche 30 bis 60 Gew.-%, bezogen auf Gesamtgemisch an Diisocyanaten der Formel

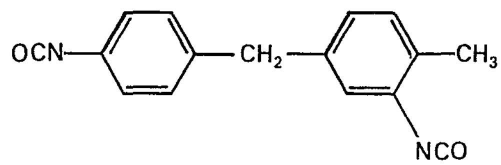

und 10 bis 40 Gew.-%, bezogen auf Gesamtgemisch, an Diisocyanaten der Formel

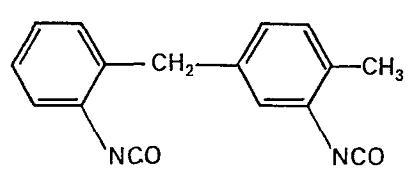

sowie 0 bis 30 Gew.-%, bezogen auf Gesamtgemisch, an anderen Methyl-substituierten Diisocyanatodiphenylmethan-Isomeren enthalten, wobei in beiden Formeln jeweils der Rest $R_2$ für eine Methylgruppe und die beiden anderen Reste jeweils für Wasserstoff stehen, dadurch gekennzeichnet, dass man

a) technische, durch Nitrierung von 4-Methylbenzylchlorid erhaltene, 10 bis 25 Gew.-%, bezogen auf Gesamtgemisch, 2-Nitro-4-methylbenzylchlorid und 75 bis 90 Gew.-%, bezogen auf Gesamtgemisch, 3-Nitro-4-methyl-benzylchlorid enthaltende Nitrobenzylchlorid-Isomerengemische in Gegenwart von Friedel-Crafts-Katalysatoren mit Benzol zu einem im wesentlichen die Mononitroverbindungen der Formeln

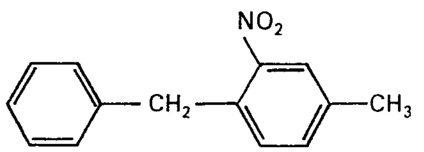

und als Hauptprodukt

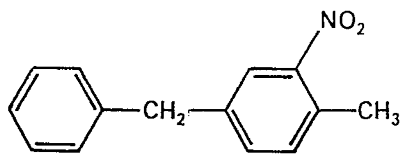

enthaltenden Friedel-Crafts-Kondensat umsetzt, den Katalysator aus dem Kondensat entfernt,

b) das gemäss a) erhaltene Kondensat einer Nitrierungsreaktion unter Bildung von im wesentlichen Nitroverbindungen der Formeln

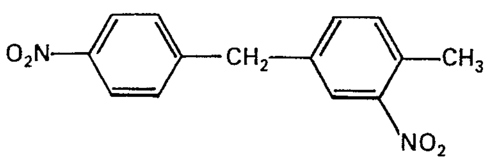

und

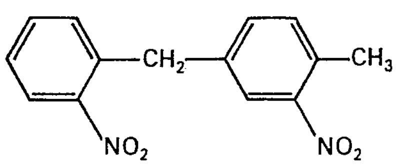

enthaltenden Gemisch aromatischer Dinitroverbindungen unterzieht,

c) die gemäss b) erhaltenen Nitroverbindungen durch Hydrierung bzw. Reduktion der Nitrogruppen in die entsprechenden aromatischen Diamine überführt,

d) die gemäss c) erhaltenen Diamine durch Phosgenierung in die entsprechenden Diisocyanate überführt,

wobei man gegebenenfalls

e) aus dem gemäss c) anfallenden Gemisch an aromatischen Aminoverbindungen vor der Phosgenierung und/oder aus dem gemäss d) erhaltenen Polyisocyanatgemisch destillativ das 30 bis 60 Gew.-%, bezogen auf Gesamtgemisch, an 3,4'-Isomeren und 10 bis 40 Gew.-%, bezogen auf Gesamtgemisch an 3,2'-Isomeren sowie 0–30 Gew.-%, bezogen auf Gesamtgemisch, an anderen methylsubstituierten Diisocyanatodiphenylmethan-Isomeren enthaltende Isomerengemisch in von Nebenprodukten befreiter Form isoliert.

6. Verfahren zur Herstellung von im Gemisch mit bis zu 40 Gew.-%, bezogen auf Gesamtgemisch, an Diisocyanaten der Formel

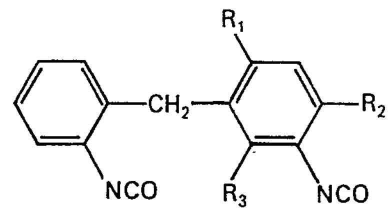

und mit bis zu 30 Gew.-%, bezogen auf Gesamtgemisch an anderen Methyl-substituierten Diisocyanatodiphenylmethan-Isomeren vorliegende Diisocyanate der Formel

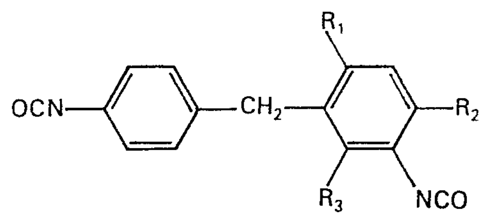

wobei in beiden Formeln jeweils einer der Reste $R_1$, $R_2$ oder $R_3$ für eine Methylgruppe und die beiden anderen Reste jeweils für Wasserstoff stehen, dadurch gekennzeichnet, dass man

a) ein Benzylhalogenid in Gegenwart von Friedel-Crafts-Katalysatoren oder Benzylalkohol in Gegenwart von Säure-Katalysatoren mit Toluol zu einem im wesentlichen aus Kohlenwasserstoffen der Formel

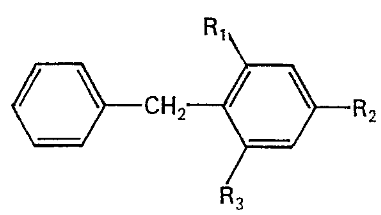

bestehenden Kondensat umsetzt,

b) das ausserdem gemäss a) erhaltenen Kondensat destillativ in reiner Form erhaltene Methyl-diphenylmethan-Isomerengemisch der zuletztgenannten Formel einer Dinitrierung unterzieht,

c) die gemäss b) erhaltenen Dinitroverbindungen durch Hydrierung bzw. Reduktion der Nitrogruppen in die entsprechenden Diamine überführt,

d) die gemäss c) erhaltenen Diamine durch Phosgenierung in die entsprechenden Diisocyanate überführt,

wobei man gegebenenfalls

e) aus den gemäss c) erhaltenen Diaminen vor der Phosgenierung und/oder aus den gemäss d) erhaltenen Diisocyanatgemischen das entsprechende im Gemisch mit bis zu 40 Gew.-% 2′,3-Isomeren und mit bis zu 30 Gew.-%, bezogen auf Gesamtgemisch, an anderen Methyl-substituierten Diisocyanatodiphenylmethan-isomeren vorliegende 3,4′-Isomere in von Nebenprodukten befreiter Form isoliert.

7. Verfahren zur Herstellung von im Gemisch mit bis zu 40 Gew.-%, bezogen auf Gesamtgemisch, an 2′,3-Diisocyanato-4-methyl-diphenylmethan und bis zu 30 Gew.-% bezogen auf Gesamtgemisch, an anderen Methyl-substituierten Diisocyanatodiphenylmethan-Isomeren vorliegenden 3,4′-Diisocyanato-4-methyl-diphenylmethan, dadurch gekennzeichnet, dass man

a) ein p-Methylbenzylhalogenid in Gegenwart von Friedel-Crafts-Katalysatoren mit Benzol zu einem aus 4-Methyl-diphenylmethan und höhermolekularen Kondensaten bestehenden Kohlenwasserstoffgemisch umsetzt,

b) das aus dem gemäss a) erhaltene Kohlenwasserstoffgemisch destillativ in reiner Form erhaltene 4-Methyl-diphenylmethan dinitriert,

c) die gemäss b) erhaltene Dinitroverbindung durch Hydrierung oder Reduktion der Nitrogruppen in die entsprechende aromatische Diamonoverbindung überführt,

d) die gemäss c) erhaltenen Diamine durch Phosgenierung in die entsprechenden Diisocyanate überführt,

wobei man gegebenenfalls

e) aus den gemäss c) erhaltenen Diaminen vor der Phosgenierungsreaktion und/oder aus den gemäss d) erhaltenen Diisocyanaten die entsprechenden im Gemisch mit bis zu 30 Gew.-%, bezogen auf Gesamtgemisch, an 2′,3-Isomeren und bis zu 30 Gew.-%, bezogen auf Gesamtgemisch, an anderen Methyl-substituierten Diisocyanatodiphenylmethan-Isomeren vorliegenden 3,4′-Isomeren in von Nebenprodukten befreiter Form isoliert.

8. Verwendung der gemäss Anspruch 1 bis 7 erhältlichen Diisocyanate bzw. Diisocyanatgemische als Isocyanatkomponente bei der Herstellung von Polyurethankunststoffen nach dem Isocyanat-Polyadditionsverfahren.

**Claims for the Contracting States: BE, CH, LI, DE, FR, GB, IT, NL, SE**

1. Diisocyanates of the formula

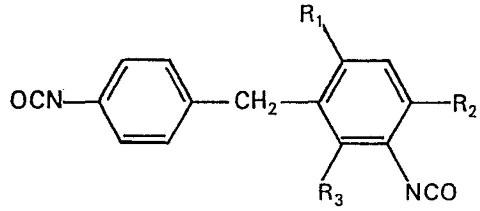

optionally in admixture with up to 40% by weight, based on the total mixture, of diisocyanates of the formula

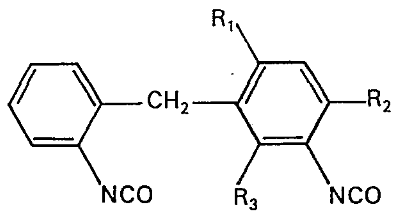

and optionally with up to 30% by weight, based on the total mixture, of other, diisocyanatodiphenyl methane isomers which may be substituted by methyl; in these formulae, the radicals $R_1$, $R_2$ and $R_3$ are the same or different and represent hydrogen or a methyl group, with the proviso that, in each of the two formulae, at least two of the radicals $R_1$, $R_2$ and $R_3$ represent hydrogen.

2. Diisocyanates or diisocyanate mixtures according to Claim 1, characterised in that, in the two formulae given in Claim 1, one of the radicals $R_1$, $R_2$ or $R_3$ represents a methyl group.

3. A diisocyanate according to Claim 1 of the formula

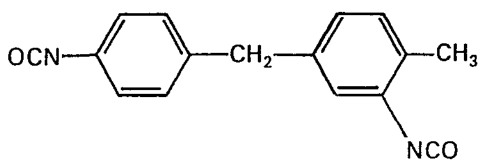

4. A diisocyanate according to Claim 1 of the formula

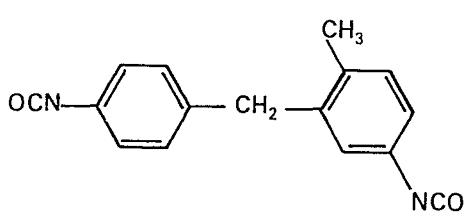

5. A process for the production of diisocyanates of the formula

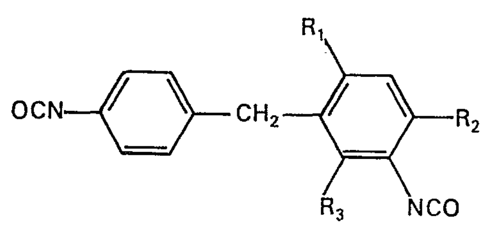

in which $R_1$, $R_2$ and $R_3$ have the meaning indicated in Claim 1, characterised in that

a) a 4-nitrobenzyl halide is reacted with nitrobenzene and/or o-nitrotoluene and/or p-nitrotoluene in the presence of Friedel-Crafts catalysts to form dinitro compounds of the formula

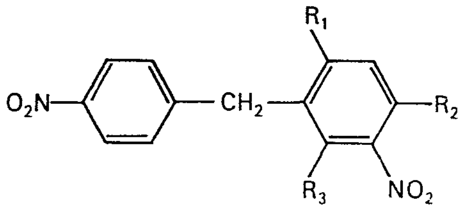

and the reaction product is freed from the catalyst used,

b) the reaction product obtained in stage a) is converted by hydrogenation or reduction of the nitro groups into the corresponding aromatic diamino compounds and

c) the diamino compounds obtained in stage b) are converted by phosgenation into the diisocyanates,

d) the corresponding 3,4'-diamino or 3,4'-diisocyanato isomers optionally being isolated free from secondary products by distillation from the diamines obtained in accordance with b) before the phosgenation reaction and/or from the diisocyanates obtained in accordance with c).

6. A process for the production of diisocyanates of the formula

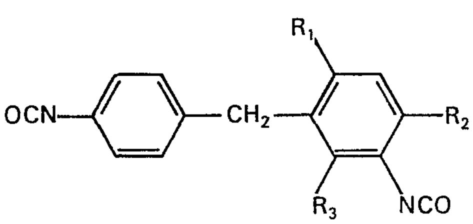

in which one of the radicals $R_1$, $R_2$ or $R_3$ represents a methyl group and the other two represent hydrogen, characterised in that

a) a 4-nitrobenzyl halide is reacted with toluene in the presence of Friedel-Crafts catalysts to form a mixture of mononitro compounds of the formula

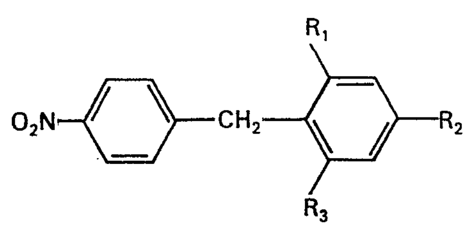

and the reaction product is freed from the catalyst,

b) the reaction product obtained in stage a) is subjected to a nitration reaction to form dinitro compounds of the formula

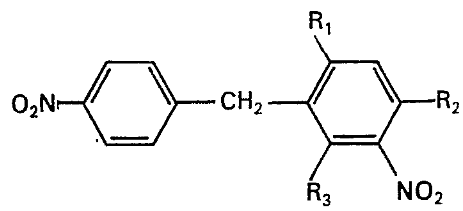

c) the dinitro compounds obtained in stage b) are converted by hydrogenation or reduction of the amino groups into the corresponding aromatic diamino compounds and

d) the diamino compounds obtained in stage c) are converted by phosgenation into the corresponding diisocyanates,

e) the corresponding 3,4'-diamino or 3,4'-diisocyanato isomers optionally being isolated free from secondary products by distillation from the diamines obtained in stage c) before the phosgenation reaction and/or from the diisocyanates obtained in stage d).

7. A process for the production of 3,4'-diisocyanatodiphenyl methan which may be present in admixture with up to 40% by weight, based on the total mixture, of 2',3-diisocyanatodiphenyl methane, characterised in that

a) a 3-nitrobenzyl halide is reacted with benzene in the presence of Friedel-Crafts catalysts to form 3-nitro diphenyl methane and the catalyst is removed from the reaction product,

b) the nitro compound obtained in a) is subjected to a nitration reaction to form 3,4'-dinitrodiphenyl methane containing up to 40% by weight of 2',3-dinitrodiphenyl methane,

c) the dinitro compounds obtained in b) are converted by hydrogenation or reduction of the nitro groups into the corresponding aromatic diamino compounds,

d) the diamino compounds obtained in c) are converted by phosgenation into the corresponding diisocyanates,

e) the pure 3,4'-isomer or mixtures thereof with up to 40% by weight of 3,2'-isomers optionally being isolated in pure form by distillation from the amino compounds obtained in c) before their phosgenation or from the polyisocyanates obtained in d).

8. A process for the production of diiscocyanatodiphenyl methane isomer mixtures containing from 30 to 60% by weight, based on the total mixture, of diisocyanates of the formula

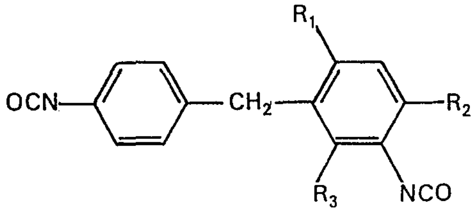

and from 10 to 40% by weight, based on the total mixture, of diisocyanates of the formula

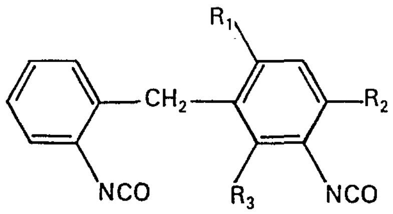

and from 0 to 30% by weight, based on the mixture as a whole, of other methyl-substituted diisocyanatodiphenyl methane isomers; in both formulae, one of the radicals $R_1$, $R_2$ or $R_3$ represent a methyl group and the other two hydrogen, characterised in that

a) commercial nitrobenzyl chloride isomer mixtures obtained by nitrating benzyl chloride and containing from 10 to 50% by weight, based on the total mixture, of 2-nitro benzyl chloride and from 50 to 90% by weight, based on the total mixture, of 4-nitrobenzyl chloride in addition to minor quantities of 3-nitrobenzyl chloride are reacted with toluene in the presence of Friedel-Crafts catalysts to form a Friedel-Crafts condensate essentially containing mononitro compounds of the formulae

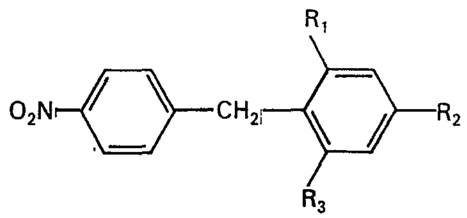

and

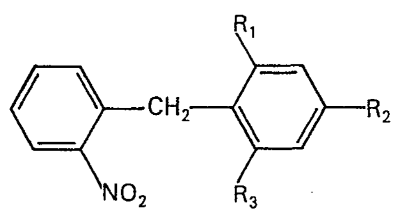

and the catalyst is removed from the condensate,

b) the condensate obtained in a) is subjected to a nitration reaction to form a mixture of aromatic dinitro compounds essentially containing nitro compounds of the formulae

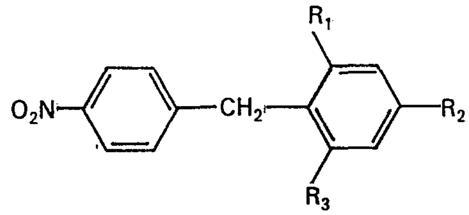

and

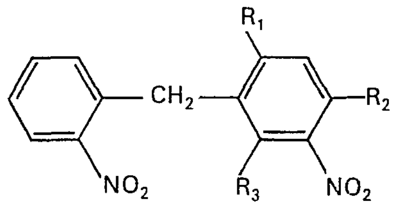

c) the nitro compounds obtained in b) are converted by hydrogenation or reduction of the nitro groups into the corresponding aromatic diamines,

d) the diamines obtained in c) are converted by phosgenation into the corresponding diisocyanates,

e) the isomer mixture containing from 30 to 60% by weight, based on the total mixture, of 3,4′-isomers and from 10 to 40% by weight, based on the total mixture, of 3,2′-isomers and from 0 to 30% by weight, based on the total mixture, of other methyl-substituted diisocyanato diphenyl methane isomers optionally being isolated free from secondary products by distillation from the mixture of aromatic amino compounds obtained in c) before the phosgenation reaction and/or from the polyisocyanate mixture obtained in d).

9. A process for the production of diisocyanatodiphenyl methane isomer mixtures containing from 30 to 60% by weight, based on the total mixture, of diisocyanates of the formula

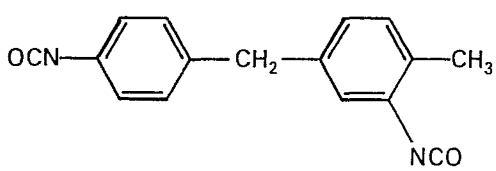

and from 10 to 40% by weight, based on the total mixture, of diisocyanates of the formula

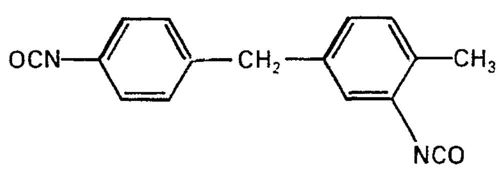

and from 0 to 30% by weight, based on the total mixture, of other methyl-substituted diisocyanato-diphenyl methane isomers; in both formulae,

the radical $R_2$ represents a methyl group and the other two radicals represent hydrogen, characterised in that

a) technical nitrobenzyl chloride isomer mixtures obtained by nitrating 4-methyl benzyl chloride and containing from 10 to 25% by weight, based on the total mixture, of 2-nitro-4-methyl benzyl chloride and from 75 to 90% by weight, based on the total mixture, of 3-nitro-4-methyl benzyl chloride are reacted with benzene in the presence of Friedel-Crafts catalysts to form a Friedel-Crafts condensate essentially containing mononitro compounds of the formulae

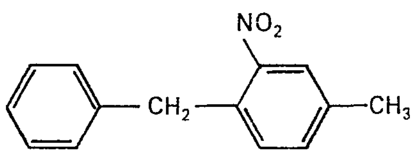

and, as main product,

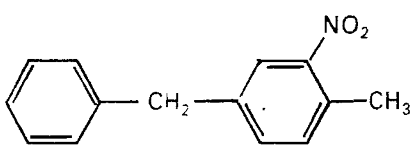

and the catalyst is removed from the condensate,

b) the condensate obtained in a) is subjected to a nitration reaction to form a mixture of aromatic dinitro compounds essentially containing nitro compounds of the formulae

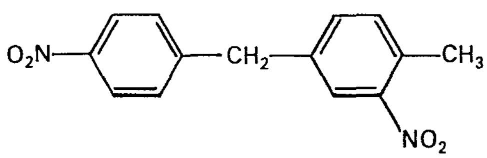

and

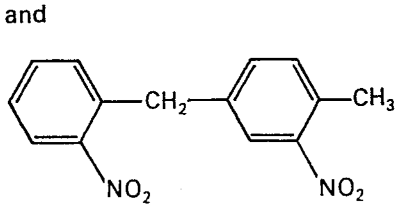

c) the nitro compounds obtained in b) are converted by hydrogenation or reduction of the nitro groups into the corresponding aromatic diamines,

d) the diamines obtained in c) are converted by phosgenation into the corresponding diisocyanates,

e) the isomer mixture containing from 30 to 60% by weight, based on the total mixture, of 3,4'-isomers and from 10 to 40% by weight, based on the total mixture, of 3,2'-isomers and from 0 to 30% by weight, based on the total mixture, of other methyl-substituted diisocyanato diphenyl methane isomers optionally being isolated free from secondary products by distillation from the mixture of aromatic amino compounds obtained in c) before the phosgenation reaction and/or from the polyisocyanate mixture obtained in d).

10. A process for the production of diisocyanates of the formula

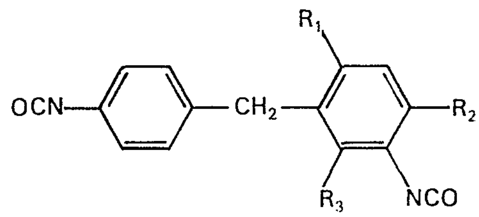

which are present in admixture with up to 40% by weight, based on the mixture, of diisocyanates of the formula

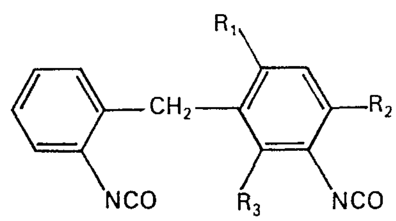

and with up to 30% by weight, based on the total mixture, of other methyl-substituted diisocyanatodiphenyl methane isomers; in both formulae, one of the radicals $R_1$, $R_2$ or $R_3$ represents a methyl group and the other radicals represent hydrogen, characterised in that

a) a benzyl halide is reacted with toluene in the presence of Friedel-Crafts catalysator benzyl alcohol is reacted with toluene in the presence of acid catalysts to form a condensate essentially consisting of hydrocarbons of the formula

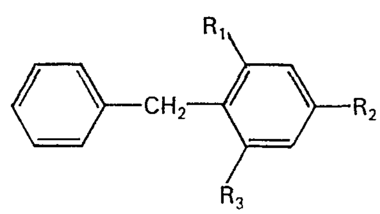

b) the methyl diphenyl methane isomer mixture corresponding to the last formula which is obtained in pure form by distillation from the condensate obtained in a) is subjected to a dinitration reaction,

c) the dinitro compounds obtained in b) are converted by hydrogenation or reduction of the nitro groups into the corresponding diamines,

d) the diamines obtained in c) are converted by phosgenation into the corresponding diisocyanates,

e) the corresponding 3,4'-isomer present in admixture with up to 40% by weight of 2',3-isomers and with up to 30% by weight, based on the total mixture, of other methyl-substituted diisocyanatodiphenyl methane isomers optionally being isolated free from secondary products from the diamines obtained in c) before the phosgenation reaction and/or from the diisocyanate mixtures obtained in d).

11. A process for the production of 3,4'-diisocyanato-4-methyl diphenyl methane present in admixture with up to 40% by weight, based on the total mixture, of 2',3-diisocyanato-4-methyl diphenyl methane and with up to 30% by weight, based on the total mixture, of other methyl-substituted diisocyanato diphenyl methane isomers, characterised in that

a) a p-methyl benzyl halide is reacted with benzene in the presence of Friedel-Crafts catalysts to form a hydrocarbon mixture consisting of 4-methyl diphenyl methane and relatively high molecular weight condensates,

b) the 4-methyl diphenyl methane obtained in pure form by distillation from the hydrocarbon mixture obtained in a) is dinitrated,

c) the dinitro compound obtained in b) is converted by hydrogenation or reduction of the nitro groups into the corresponding aromatic diamino compound,

d) the diamines obtained in c) are converted by phosgenation into the corresponding diisocyanates,

e) the corresponding 3,4'-isomers present in admixture with up to 30% by weight, based on the total mixture, of 2',3-isomers and with up to 30% by weight, based on the total mixture, of other methyl-substituted diisocyanato diphenyl methane isomers optionally being isolated free from secondary products from the diamines obtained in c) before the phosgenation reaction and/or from the diisocyanates obtained in d).

12. The use of the diisocyanates or diisocyanate mixtures according to claims 1 to 4 as isocyanate component in the production of polyurethan plastics by the isocyanate polyaddition process.

**Claims for the Contracting State: AT**

1. A process for the production of diisocyanates of the formula

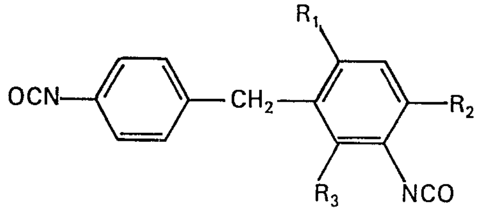

in which

$R_1$, $R_2$ and $R_3$ are the same or different and represent hydrogen or a methyl group, with the proviso that, in each of the two formulae, at least two of the radicals $R_1$, $R_2$ and $R_3$ represent hydrogen, characterised in that

a) a 4-nitrobenzyl halide is reacted with nitrobenezene and/or o-nitrotoluene and/or p-nitrotoluene in the presence of Friedel-Crafts catalysts to form dinitro compounds of the formula

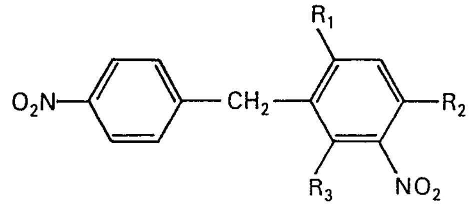

and the reaction product is freed from the catalyst used,

b) the reaction product obtained in stage a) is converted by hydrogenation or reduction of the nitro groups into the corresponding aromatic diamino compounds and

c) the diamino compounds obtained in stage b) are converted by phosgenation into the diisocyanates,

d) the corresponding 3,4'-diamino or 3,4'-diisocyanato isomers optionally being isolated free from secondary products by distillation from the diamines obtained in accordance with b) before the phosgenation reaction and/or from the diisocyanates obtained in accordance with c).

2. A process for the production of diisocyanates of the formula

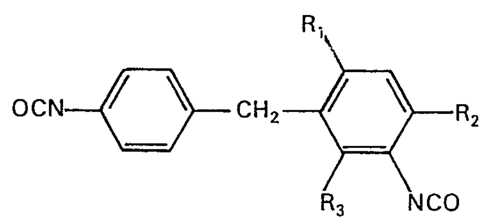

in which one of the radicals $R_1$, $R_2$ or $R_3$ represents a methyl group and the other two represent hydrogen, characterised in that

a) a 4-nitrobenzyl halide is reacted with toluene in the presence of Friedel-Crafts catalysts to form a mixture of mononitro compounds of the formula

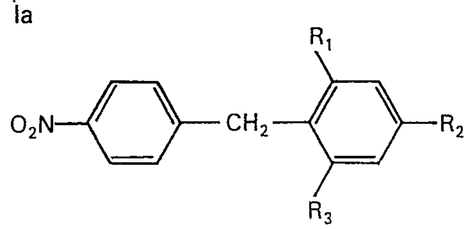

and the reaction product is freed from the catalyst,

b) the reaction product obtained in stage a) is subjected to a nitration reaction to form dinitro compounds of the formula

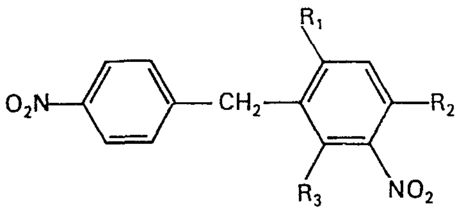

c) the dinitro compounds obtained in stage b) are converted by hydrogenation or reduction of the amino groups into the corresponding aromatic diamino compounds and

d) the diamino compounds obtained in stage c) are converted by phosgenation into the corresponding diisocyanates,

e) the corresponding 3,4′-diamino or 3,4′-diisocyanato isomers optionally being isolated free from secondary products by distillation from the diamines obtained in stage c) before the phosgenation reaction and/or from the diisocyanates obtained in stage d).

3. A process for the production of 3,4′-diisocyanatodiphenyl methan which may be present in admixture with up to 40% by weight, based on the total mixture, of 2′,3-diisocyanatodiphenyl methane, characterised in that

a) a 3-nitrobenzyl halide is reacted with benzene in the presence of Friedel-Crafts catalysts to form 3-nitro diphenyl methane and the catalyst is removed from the reaction product,

b) the nitro compound obtained in a) is subjected to a nitration reaction to form 3,4′-dinitrodiphenyl methane containing up to 40% by weight of 2′,3-dinitrodiphenyl methane,

c) the dinitro compounds obtained in b) are converted by hydrogenation or reduction of the nitro groups into the corresponding aromatic diamino compounds,

d) the diamino compounds obtained in c) are converted by phosgenation into the corresponding diisocyanates,

e) the pure 3,4′-isomer or mixture thereof with up to 40% by weight of 3,2′-isomers optionally being isolated in pure form by distillation from the amino compounds obtained in c) before their phosgenation or from the polyisocyanates obtained in d).

4. A process for the production of diisocyanatodiphenyl methane isomer mixtures containing from 30 to 60% by weight, based on the total mixture, of diisocyanates of the formula

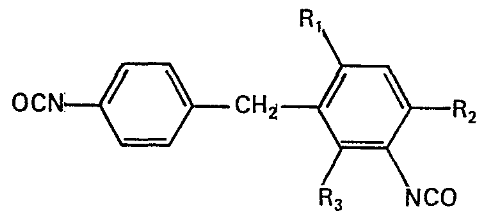

and from 10 to 40% by weight, based on the total mixture, of diisocyanates of the formula

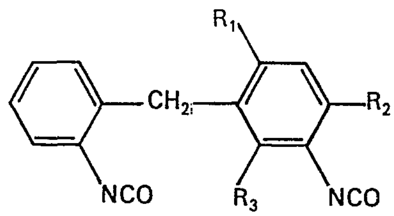

and from 0 to 30% by weight, based on the mixture as a whole, of other methyl-substituted diisocyanatodiphenyl methane isomers; in both formulae, one of the radicals $R_1$, $R_2$ or $R_3$ represent a methyl group and the other two hydrogen, characterised in that

a) commercial nitrobenzyl chloride isomer mixtures obtained by nitrating benzyl chloride and containing from 10 to 50% by weight, based on the total mixture, of 2-nitro benzyl chloride and from 50 to 90% by weight, based on the total mixture, of 4-nitrobenzyl chloride in addition to minor quantities of 3-nitrobenzyl chloride are reacted with toluene in the presence of Friedel-Crafts catalysts to form a Friedel-Crafts condensate essentially containing mononitro compounds of the formulae

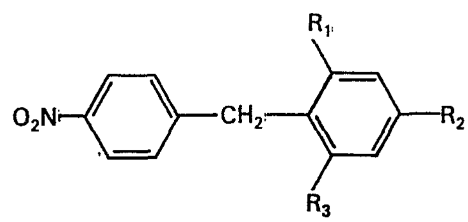

and

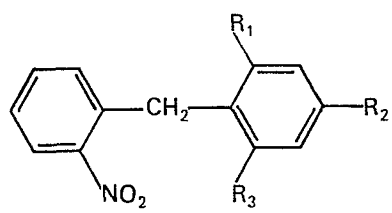

and the catalyst is removed from the condensate,

b) the condensate obtained in a) is subjected to a nitration reaction to form a mixture of aromatic dinitro compounds essentially containing nitro compounds of the formulae

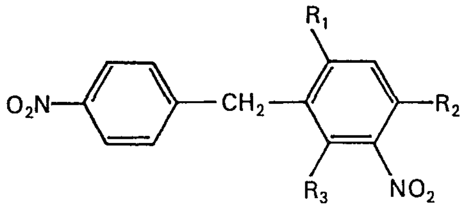

and

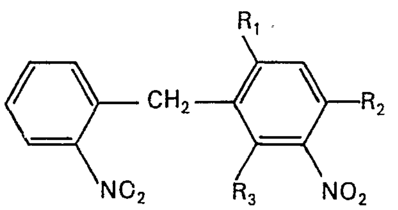

c) the nitro compounds obtained in b) are converted by hydrogenation or reduction of the nitro groups into the corresponding aromatic diamines,

d) the diamines obtained in c) are converted by phosgenation into the corresponding diisocyanates,

e) the isomer mixture containing from 30 to 60% by weight, based on the total mixture, of 3,4′-isomers and from 10 to 40% by weight, based on the total mixture, of 3,2′-isomers and from 0 to 30% by weight, based on the total mixture, of other methyl-substituted diisocyanato diphenyl methane isomers optionally being isolated free from secondary products by distillation from the mixture of aromatic amino compounds obtained in c) before the phosgenation reaction and/or from the polyisocyanate mixture obtained in d).

5. A process for the production of diisocyanatodiphenyl methane isomer mixtures containing from 30 to 60% by weight, based on the total mixture, of diisocyanates of the formula

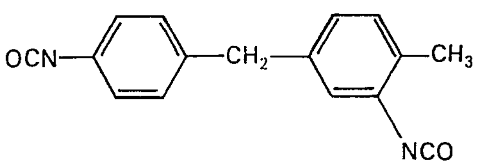

and from 10 to 40% by weight, based on the total mixture, of diisocyanates of the formula

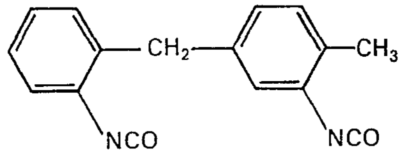

and from 0 to 30% by weight, based on the total mixture, of other methyl-substituted diisocyanatodiphenyl methane isomers; in both formulae, the radical $R_2$ represent a methyl group and the other two radicals represent hydrogen, characterised in that

a) technical nitrobenzyl chloride isomer mixtures obtained by nitrating 4-methyl benzyl chloride and containing from 10 to 25% by weight, based on the total mixture, of 2-nitro-4-methyl benzyl chloride and from 75 to 90% by weight, based on the total mixture, of 3-nitro-4-methyl benzyl chloride are reacted with benzene in the presence of Friedel-Crafts catalysts to form a Friedel-Crafts condensate essentially containing mononitro compounds of the formulae

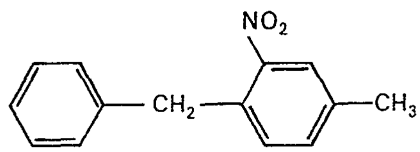

and, as main product,

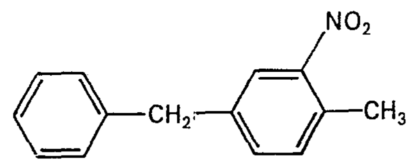

and the catalyst is removed from the condensate,

b) the condensate obtained in a) is subjected to a nitration reaction to form a mixture of aromatic dinitro compounds essentially containing nitro compounds of the formulae

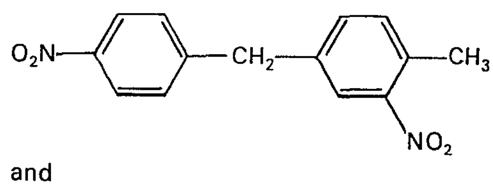

and

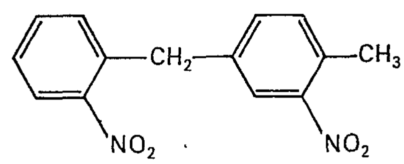

c) the nitro compounds obtained in b) are converted by hydrogenation or reduction of the nitro groups into the corresponding aromatic diamines,

d) the diamines obtained in c) are converted by phosgenation into the corresponding diisocyanates,

e) the isomer mixture containing from 30 to 60% by weight, based on the total mixture, of 3,4′-isomers and from 10 to 40% by weight, based on the total mixture, of 3,2′-isomers and from 0 to 30% by weight, based on the total mixture, of other methyl-substituted diisocyanato diphenyl methane isomers optionally being isolated free from secondary products by distillation from the mixture of aromatic amino compounds obtained in c) before the phosgenation reaction and/or from the polyisocyanate mixture obtained in d).

6. A process for the production of diisocyanates of the formula

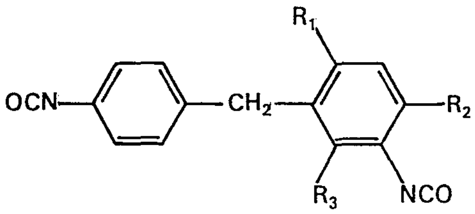

which are present in admixture with up to 40% by weight, based on the total mixture, of diisocyanates of the formula

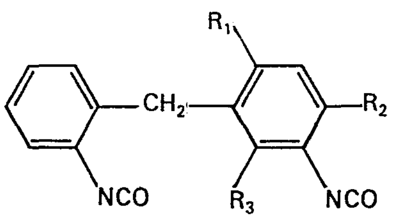

and with up to 30% by weight, based on the total mixture, of other methyl-substituted diisocyanatodiphenyl methane isomers; in both formulae, one of the radicals $R_1$, $R_2$ or $R_3$ represents a methyl group and the other two radicals represent hydrogen, characterised in that

a) a benzyl halide is reacted with toluene in the presence of Friedel-Crafts catalysts or benzyl alcohol is reacted with toluene in the presence of acid catalysts to form a condensate essentially consisting of hydrocarbons of the formula

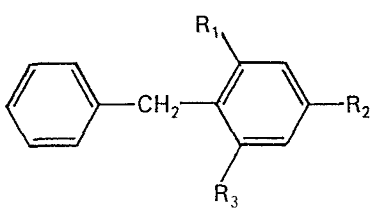

b) the methyl diphenyl methane isomer mixture corresponding to the last formula which is obtained in pure form by distillation from the condensate obtained in a) is subjected to a dinitration reaction,

c) the dinitro compounds obtained in b) are converted by hydrogenation or reduction of the nitro groups into the corresponding diamines,

d) the diamines obtained in c) are converted by phosgenation into the corresponding diisocyanates,

e) the corresponding 3,4'-isomer present in admixture with up to 40% by weight of 2'-3-isomers and with up to 30% by weight, based on the total mixture, of other methyl-substituted diisocyanatodiphenyl methane isomers optionally being isolated free from secondary products from the diamines obtained in c) before the phosgenation reaction and/or from the diisocyanate mixtures obtained in d).

7. A process for the production of 3,4'-diisocyanato-4-methyl diphenyl methane present in admixture with up to 40% by weight, based on the total mixture, of 2',3-diisocyanato-4-methyl diphenyl methane and with up to 30% by weight, based on the total mixture, of other methyl-substituted diisocyanato diphenyl methane isomers, characterised in that

a) a p-methyl halide is reacted with benzene in the presence of Friedel-Crafts catalysts to form a hydrocarbon mixture consisting of 4-methyl diphenyl methane and relatively high molecular weight condensates,

b) the 4-methyl diphenyl methane obtained in pure form by distillation from the hydrocarbon mixture obtained in a) is dinitrated,

c) the dinitro compound obtained in b) is converted by hydrogenation or reduction of the nitro groups into the corresponding aromatic diamino compound,

d) the diamines obtained in c) are converted by phosgenation into the corresponding diisocyanates,

e) the corresponding 3,4'-isomers present in admixture with up to 30% by weight, based on the total mixture, of 2',3-isomers and with up to 30% by weight, based on the total mixture, of other methyl-substituted diisocyanato diphenyl methane isomers optionally being isolated free from secondary products from the diamines obtained in c) before the phosgenation reaction and/or from the diisocyanates obtained in d).

8. The use of the diisocyanates or diisocyanate mixtures obtainable according to Claims 1 to 7 as isocyanate component in the production of polyurethane plastics by the isocyanate polyaddition process.

**Revendications pour les Etats contractants: BE, CH, LI, DE, FR, GB, IT, NL, SE**

1. Diisocyanates de formule:

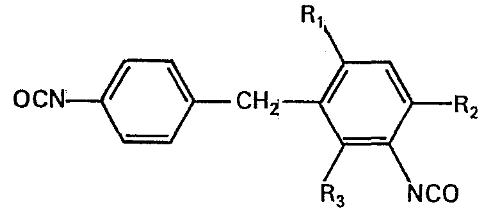

éventuellement en mélange avec jusqu'à 40% en poids, par rapport au mélange total, de diisocyanates de formule:

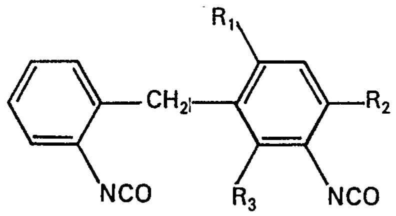

et, le cas échéant, avec jusqu'à 30% en poids, par rapport au mélange total, d'autres isomères de diisocyanatodiphénylméthane portant éventuellement des substituants méthyle, formules dans lesquelles les restes $R_1$, $R_2$ et $R_3$ sont dans chaque cas égaux ou différents et désignent de l'hydrogène ou un groupe méthyle, à condition que dans chacune des deux formules, au moins deux des restes $R_1$, $R_2$ et $R_3$ représentent dans chaque cas de l'hydrogène.

2. Diisocyanates et mélanges de diisocyanates suivant la revendication 1, caractérisés en ce que dans chacune des deux formules mentionnées dans la revendication 1, l'un des restes $R_1$, $R_2$ ou $R_3$ désigne un groupe méthyle.

3. Diisocyanate suivant la revendication 1, de formule:

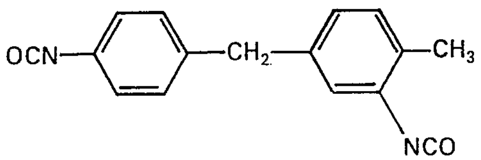

4. Diisocyanate suivant la revendication 1, de formule:

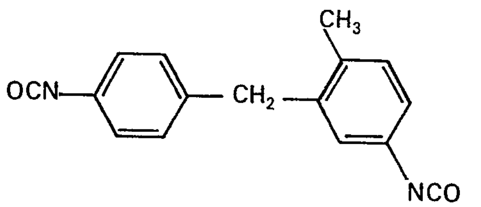

5. Procédé de production de diisocyanates de formule:

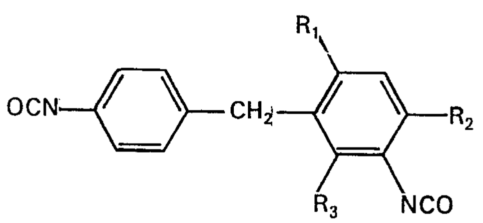

dans laquelle $R_1$, $R_2$ et $R_3$ ont la définition mentionnée dans la revendication 1, caractérisé en ce que

a) on fait réagir un halogénure de 4-nitrobenzyle en présence de catalyseurs de Friedel-Crafts avec le nitrobenzène et/ou le o-nitrotoluène et/ou le p-nitrotoluène pour former des composés dinitrés de formule:

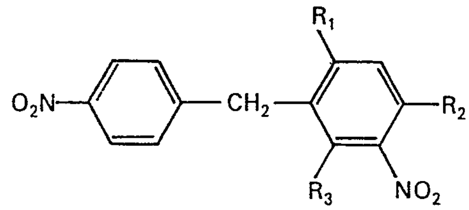

on débarrasse le produit réactionnel du catalysateur utilisé,

b) le produit de réaction obtenu conformément à a) est transformé par hydrogénation ou par réduction des groupes nitro en les composés diaminés aromatiques correspondants et

c) les composés diaminés obtenus conformément à b) sont transformés par phosgénation en les diisocyanates, et, le cas échéant,

d) on isole par distillation des diamines obtenues conformément à b) avant la réaction de phosgénation et/ou des diisocyanates obtenus conformément à c), les isomères 3,4'-diamino- et, respectivement, 3,4'-diisocyanato correspondants, sous la forme débarrassée des produits secondaires.

6. Procédé de production de diisocyanates de formule:

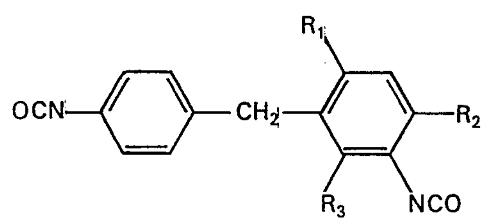

dans laquelle l'un des restes $R_1$, $R_2$ ou $R_3$ représente un groupe méthyle et les deux autres restes mentionnées représentent de l'hydrogène, caractérisé en ce que

a) on fait réagir un halogénure de 4-nitrobenzyle en présence de catalyseurs de Friedel-Crafts avec du toluène pour former un mélange de composés mononitrés de formule:

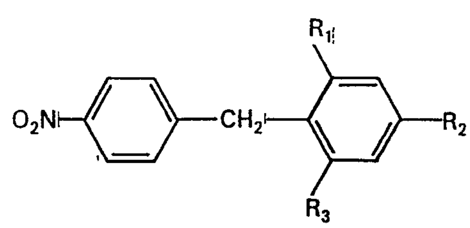

on débarrasse le produit de réaction du catalyseur,

b) on soumet le produit réactionnel obtenu conformément à a) à une réaction de nitration avec formation de composés dinitrés de formule:

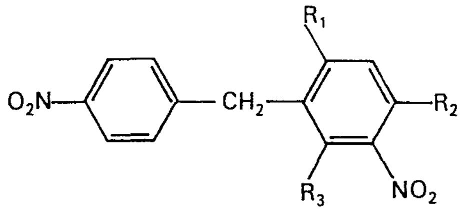

c) les composés dinitrés obtenus conformément à b) sont transformés par hydrogénation ou réduction des groupes amino en les composés diaminés aromatiques correspondants et

d) on transforme les composés diaminés obtenus conformément à c) par phosgénation en les diisocyanates correspondants, et, le cas échéant,

e) on isole par distillation des diamines obtenues conformément à c) avant la réaction de phosgénation et/ou des diisocyanates obtenus conformément à d), les isomères 3,4'-diamino- et, respectivement, 3,4'-diisocyanato correspondants, sous une forme débarrassée des sous-produits.

7. Procédé de production de 3,4'-diisocyanato-diphénylméthane se présentant éventuellement en mélange avec jusqu'à 40% en poids, par rapport au mélange total, de 2',3-diisocyanatodiphénylméthane, caractérisé en ce que

a) on fait réagir un halogénure de 3-nitrobenzyle en présence de catalyseurs de Friedel-Crafts avec du benzène pour former du 3-nitrodiphénylméthane, on élimine le catalyseur du produit réactionnel,

b) on soumet le composé nitré obtenu conformément à a) une réaction de nitration avec formation de 3,4'-dinitrodiphénylméthane contenant jusqu'à 40% en poids de 2',3-dinitrodiphénylméthane,

c) on transforme les composés dinitrés obtenus conformément à b) par hydrogénation ou réduction des groupes nitro en les composés diaminés aromatiques correspondants,

d) on transforme les composés diaminés obtenus conformément à c) par phosgénation en les diisocyanates correspondants, et, le cas échéant,

e) on isole par distillation des composés aminés obtenus conformément à c) avant leur phosgénation ou des polyisocyanates obtenus conformément à d), l'isomère 3,4' pur ou ses mélanges avec jusqu'à 40% en poids d'isomère 3,2', sous la forme pure.

8. Procédé de production de mélanges d'isomères de diisocyanatodiphénylméthane qui contiennent 30 à 60% en poids, par rapport au mélange total, de diisocyanates de formule:

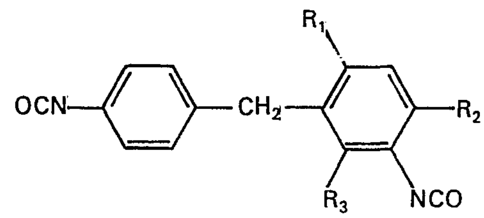

et 10 à 40% en poids, par rapport au mélange total, de diisocyanates de formule:

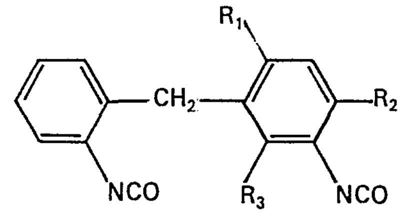

de même que 0 à 30% en poids, par rapport au mélange total, d'autres isomères de diisocyanatodiphénylméthane à substituants méthyle, l'un des restes $R_1$, $R_2$ ou $R_3$ dans chacune des deux formules représentant un groupe méthyle et les deux autres restes représentant dans chaque cas de l'hydrogène, caractérisé en ce que

a) on fait réagir des mélanges techniques d'isomères de chlorure de nitrobenzyle obtenus par nitration du chlorure de benzyle, contenant 10 à 50% en poids, par rapport au mélange total, de chlorure de 2-nitrobenzyle et 50 à 90% en poids, par rapport au mélange total, de chlorure de 4-nitro-benzyle à côté de faibles quantités de chlorure de 3-nitrobenzyle, en présence de catalysateurs de Friedel-Crafts, avec du toluène pour former un produit de condensation de Friedel-Crafts contenant principalement des composés mononitrés de formules:

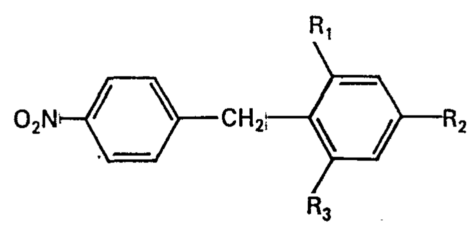

et

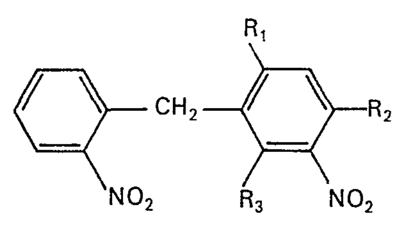

on élimine le catalyseur du produit de condensation,

b) on soumet le produit de condensation obtenu conformément à a) à une réaction de nitration avec formation d'un mélange de composés aromatiques dinitrés contenant principalement des composés nitrés de formules:

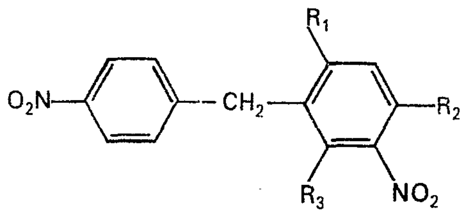

et

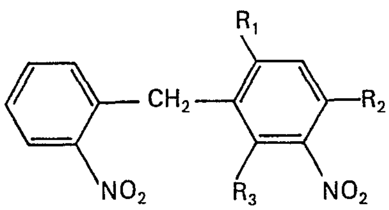

c) on transforme les composés nitrés obtenus conformément à b) par hydrogénation ou réduction des groupes nitro en les diamines aromatiques correspondantes,

d) on transforme les diamines obtenues conformément à c) par phosgénation en les diisocyanates correspondants, et, le cas échéant,

e) on isole par distillation du mélange, obtenu conformément à c), de composés aminés aromatiques avant la phosgénation et/ou du mélange de polyisocyanates obtenu conformément à d), le mélange d'isomères contenant 30 à 60% en poids, par rapport au mélange total, d'isomères 3,4′ et 10 à 40% en poids, par rapport au mélange total, d'isomères 3,2′ ainsi que 0 à 30% en poids, par rapport au mélange total, d'autres isomères de diisocyanatodiphénylméthane à substituants méthyle, sous une forme débarrassée des sous-produits.

9. Procédé de production de mélanges d'isomères de diisocyanatodiphénylméthane, qui contiennent 30 à 60% en poids, par rapport au mélange total, de diisocyanates de formule:

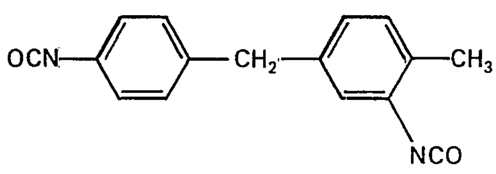

et 10 à 40% en poids, par rapport au mélange total, de diisocyanates de formule:

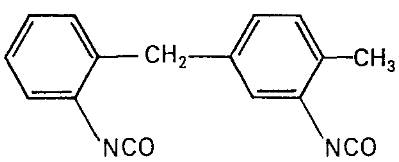

ainsi que 0 à 30% en poids, par rapport au mélange total, d'autres isomères de diisocyanatodiphénylméthane à substituants méthyle, le reste $R_2$ dans chacune des deux formules représentant un groupe méthyle et les deux autres restes représentant dans chaque cas de l'hydrogène, caractérisé en ce que:

a) on fait réagir des mélanges techniques d'isomères de chlorure de nitrobenzyle obtenus par nitration du chlorure de 4-méthylbenzyle, contenant 10 à 25% en poids, par rapport au mélange total, de chlorure de 2-nitro-4-méthylbenzyle et 75 à 90% en poids, par rapport au mélange total, de chlorure de 3-nitro-4-méthylbenzyle, en présence de catalyseurs de Friedel-Crafts avec du benzène pour former un produit de condensation de Friedel-Crafts contenant principalement les composés mononitrés de formules:

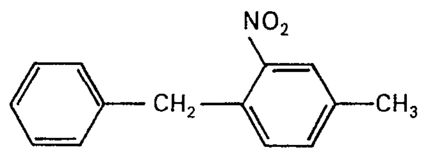

et comme produit principal

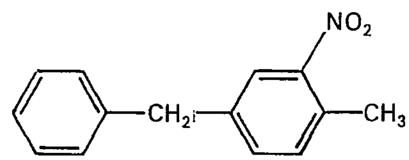

on élimine le catalysateur du produit de condensation,

b) on soumet le produit de condensation obtenu conformément à a) à une réaction de nitration avec formation d'un mélange de composés dinitrés aromatiques contenant principalement des composés nitrés de formules:

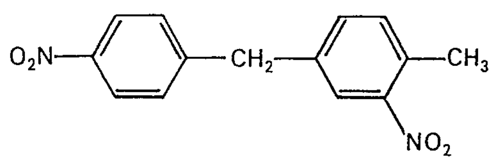

et

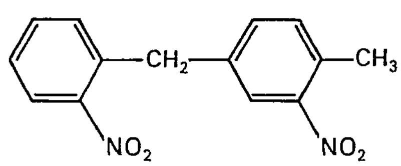

c) on transforme les composés nitrés obtenus conformément à b) par hydrogénation ou réduction des groupes nitro en les diamines aromatiques correspondantes,

d) on transforme les diamines obtenues conformément à c) par phosgénation en les diisocyanates correspondants, et, le cas échéant,

e) on isole par distillation du mélange, obtenu conformément à c), de composés aminés aromatiques avant la phosgénation et/ou du mélange, obtenu conformément à d), de polyisocyanates,

le mélange d'isomères contenant 30 à 60% en poids, par rapport au mélange total, d'isomères 3,4′ et 10 à 40% en poids, par rapport au mélange total, d'isomères 3,2′, ainsi que 0 à 30% en poids, par rapport au mélange total, d'autres isomères de diisocyanatodiphénylméthane portant des substituants méthyle, sous une forme débarrassée des sous-produits.

10. Procédé de production de diisocyanates de formule:

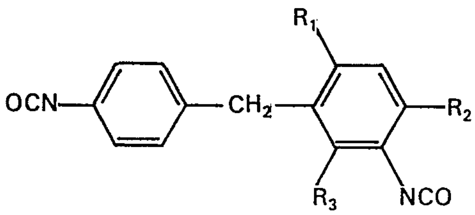

présents en mélange avec jusqu'à 40% en poids, par rapport au mélange total, de diisocyanates de formule:

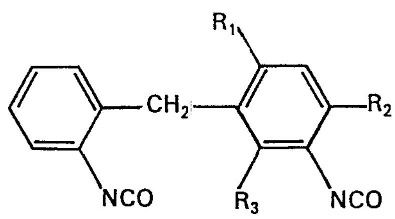

et avec jusqu'à 30% en poids, par rapport au mélange total, d'autres isomères de diisocyanatodiphénylméthane à substituants méthyle, l'un des restes $R_1$, $R_2$ ou $R_3$ dans chacune des deux formules désignant un reste méthyle et les deux autres restes désignant dans chaque cas de l'hydrogène, caractérisé en ce que

a) on fait réagir un halogénure de benzyle en présence de catalyseurs de Friedel-Crafts ou l'alcool benzylique en présence de catalyseurs acides avec le toluène pour former un produit de condensation principalement composé d'hydrocarbures de formule:

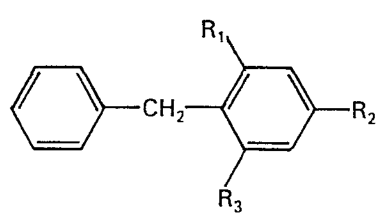

b) on soumet à une dinitration le mélange d'isomères de méthyldiphénylméthane de la formule mentionné en dernier lieu, obtenu par distillation sous la forme pure à partir du (en outre) produit de condensation obtenu conformément à a),

c) on transforme les composés dinitrés obtenus conformément à b) par hydrogénation ou réduction des groupes nitro en les diamines correspondantes,

d) on transforme les diamines obtenues conformément à c) par phosgénation en les diisocyanates correspondants, et, le cas échéant,

e) on isole des diamines obtenues conformément à c) avant la phosgénation et/ou des mélanges de diisocyanates obtenus conformément à d), l'isomère 3,4′ correspondant se présentant en mélange avec jusqu'à 40% en poids d'isomères 2′,3 et avec jusqu'à 30% en poids, par rapport au mélange total, d'autres isomères de diisocyanatodiphénylméthane à substituants méthyle, sous une forme débarrassée des sous-produits.

11. Procédé de production de 3,4′-diisocyanato-4-méthyldiphénylméthane se présentant en mélange avec jusqu'à 40% en poids, par rapport au mélange total, de 2′,3-diisocyanato-4-méthyldiphénylméthane et jusqu'à 30% en poids, par rapport au mélange total, d'autres isomères de diisocyanatodiphénylméthane à substituants méthyle, caractérisé en ce que

a) on fait réagir un halogénure de p-méthylbenzyle en présence de catalyseurs de Friedel-Crafts avec du benzène pour former un mélange d'hydrocarbures composé de 4-méthyl-diphénylméthane et de condensats de plus haut poids moléculaire,

b) on effectue la dinitration du 4-méthyldiphénylméthane obtenu par distillation sous la forme pure à partir du mélange d'hydrocarbures obtenu conformément à a),

c) on transforme le composé dinitré obtenu conformément à b) par hydrogénation ou réduction des groupes nitro en le composé diamino aromatique correspondant,

d) on transforme les diamines obtenues conformément à c) par phosgénation en les diisocyanates correspondants, et, le cas échéant,

e) on isole des diamines obtenues conformément à c) avant la réaction de phosgénation et/ou des diisocyanates obtenus conformément à d), les isomères 3,4′ correspondants en mélange avec jusqu'à 30% en poids, par rapport au mélange total, d'isomères 2′,3 et jusqu'à 30% en poids, par rapport au mélange total, d'autres isomères de diisocyanatodiphényl-méthane à substituants méthyle, sous une forme débarrassée des sous-produits.

12. Utilisation des diisocyanates et mélanges de diisocyanates suivant les revendications 1 à 4 comme composant isocyanate dans la production de matières plastiques du type polyuréthanne par le procédé de polyaddition d'un isocyanate.

**Revendications pour l'Etat contractant: AT**

1. Procédé de production de diisocyanates de formule:

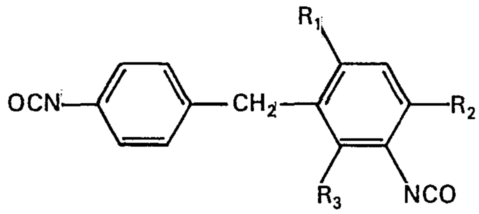

dans laquelle $R_1$, $R_2$ et $R_3$ sont dans chaque cas égaux ou différents et désignent de l'hydrogène ou un groupe méthyle, à condition que dans chacune des deux formules, au moins deux des restes $R_1$, $R_2$ et $R_3$ représentent dans chaque cas de l'hydrogène, caractérisé en ce que

a) on fait réagir un halogénure de 4-nitrobenzyle en présence de catalyseurs de Friedel-Crafts avec le nitrobenzène et/ou le o-nitrotoluène et/ou le p-nitrotoluène pour former des composés dinitrés de formule:

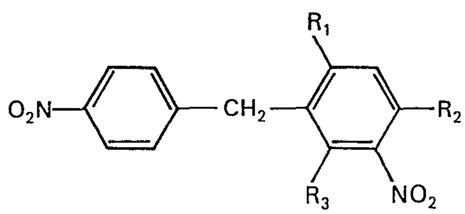

on débarrasse le produit réactionnel du catalyseur utilisé,

b) le produit de réaction obtenu conformément à a) est transformé par hydrogénation ou par réduction des groupes nitro en les composés diaminés aromatiques correspondants et

c) les composés diaminés obtenus conformément à b) sont transformés par phosgénation en les diisocyanates, et, le cas échéant,

d) on isole par distillation des diamines obtenues conformément à b) avant réaction de phosgénation et/ou des diisocyanates obtenus conformément à c), les isomères 3,4'-diamino- et, respectivement, 3,4'-diisocyanato correspondants, sous la forme débarrassée des produits secondaires.

2. Procédé de production de diisocyanates de formule:

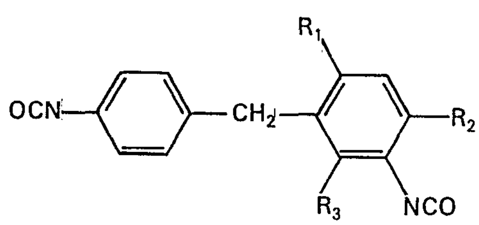

dans laquelle l'un des restes $R_1$, $R_2$ ou $R_3$ représente un groupe méthyle et les deux autres mentionnés représentent de l'hydrogène, caractérisé en ce que

a) on fait réagir un halogénure de 4-nitrobenzyle en présence de catalyseurs de Friedel-Crafts avec du toluène pour former un mélange de composés mononitrés de formule:

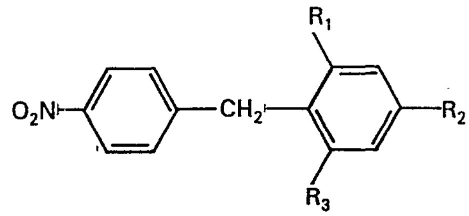

on débarrasse le produit de réaction du catalyseur,

b) on soumet le produit réactionnel obtenu conformément à a) à une réaction de nitration avec formation de composés dinitrés de formule:

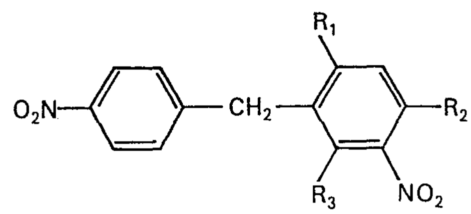

c) les composés dinitrés obtenus conformément à b) sont transformés par hydrogénation ou réduction des groupes amino en les composés diaminés aromatiques correspondants et

d) on transforme les composés diaminés obtenus conformément à c) par phosgénation en les diisocyanates correspondants, et, le cas échéant,

e) on isole par distillation des diamines obtenues conformément à c) avant la réaction de phosgénation et/ou des diisocyanates obtenus conformément à d), les isomères 3,4'-diamino- et, respectivement, 3,4'-diisocyanato correspondants, sous une forme débarrassée des sous-produits.

3. Procédé de production de 3,4'-diisocyanatodiphénylméthane se présentant éventuellement en mélange avec jusqu'à 40% en poids, par rapport au mélange total, de 2',3-diisocyanatodiphénylméthane, caractérisé en ce que

a) on fait réagir un halogénure de 3-nitrobenzyle en présence de catalyseurs de Friedel-Crafts avec du benzène pour former du 3-nitrodiphénylméthane, on élimine le catalyseur du produit réactionnel,

b) on soumet le composés nitré obtenu conformément à a) à une réaction de nitration avec formation de 3,4'-dinitrodiphénylméthane contenant jusqu'à 40% en poids de 2'-3-dinitrodiphénylméthane,

c) on transforme les composés dinitrés obtenus conformément à b) par hydrogénation ou réduction des groupes nitro en les composés diamines aromatiques correspondants,

d) on transforme les composés diaminés obtenus conformément à c) par phosgénation en les diisocyanates correspondants, et, le cas échéant,

e) on isole par distillation des composés aminés obtenus conformément à c) avant leur phosgénation ou des polyisocyanates obtenus conformément à d), l'isomère 3,4' pur ou ses mélanges avec jusqu'à 40% en poids d'isomère 3,2', sous la forme pure.

4. Procédé de production de mélanges d'isomères de diisocyanatodiphénylméthane qui contiennent 30 à 60% en poids, par rapport au mélange total, de diisocyanates de formule:

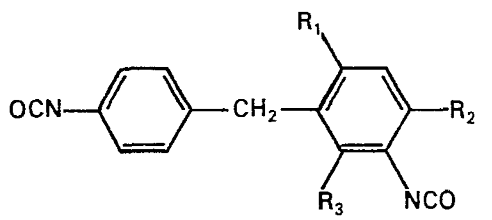

et 10 à 40% en poids, par rapport au mélange total, de diisocyanates de formule:

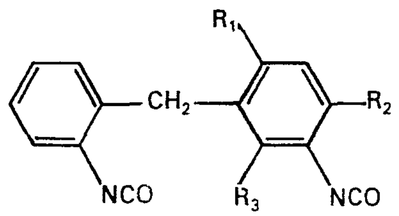

de même que 0 à 30% en poids, par rapport au mélange total, d'autres isomères de diisocyanatodiphénylméthane à substituants méthyle, l'un des restes $R_1$, $R_2$ ou $R_3$ dans chacune des deux formules représentant un groupe méthyle et les deux autres restes représentant dans chaque cas de l'hydrogène, caractérisé en ce que

a) on fait réagir des mélanges techniques d'isomères de chlorure de nitrobenzyle obtenus par nitration du chlorure de benzyle, contenant 10 à 50% en poids, par rapport au mélange total, de chlorure de 2-nitrobenzyle et 50 à 90% en poids, par rapport au mélange total, de chlorure de 4-nitrobenzyle à côté de faibles quantités de chlorure de 3-nitrobenzyle, en présence de catalyseurs de Friedel-Crafts, avec du toluène pour former un produit de condensation de Friedel-Crafts contenant principalement des composés mononitrés de formules:

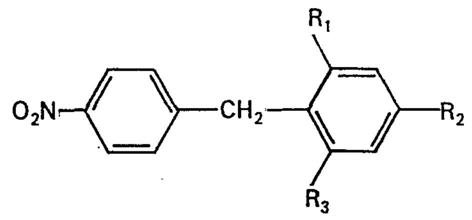

et

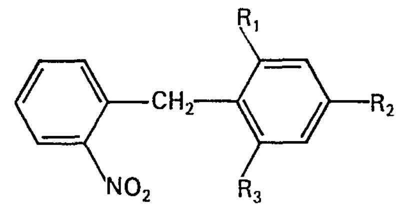

on élimine le catalyseur du produit de condensation,

b) on soumet le produit de condensation obtenu conformément à a) à une réaction de nitration avec formation d'un mélange de composés aromatiques dinitrés contenant principalement des composés nitrés de formules:

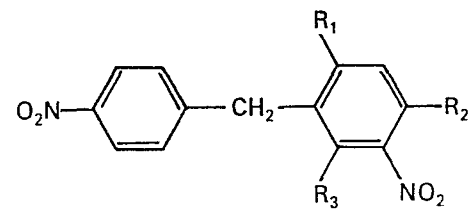

et

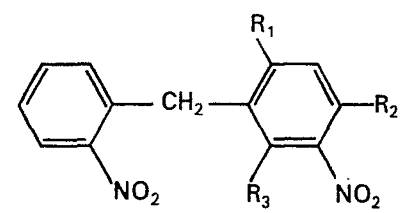

c) on transforme les composés nitrés obtenus conformément à b) par hydrogénation ou réduction des groupes nitro en les diamines aromatiques correspondantes,

d) on transforme les diamines obtenues conformément à c) par phosgénation en les diisocyanates correspondants, et, le cas échéant,

e) on isole par distillation du mélange, obtenu conformément à c), de composés aminés aromatiques avant la phosgénation et/ou du mélange de polyisocyanates obtenu conformément à d), le mélange d'isomères contenant 30 à 60% en poids, par rapport au mélange total, d'isomères 3,4' et 10 à 40% en poids, par rapport au mélange total, d'isomères 3,2' ainsi que 0 à 30% en poids, par rapport au mélange total, d'autres isomères de diisocyanatodiphénylméthane à substituants méthyle, sous une forme débarrassée des sous-produits.

5. Procédé de production de mélanges d'isomères de diisocyanatodiphénylméthane, qui contiennent 30 à 60% en poids, par rapport au mélange total, de diisocyanates de formule:

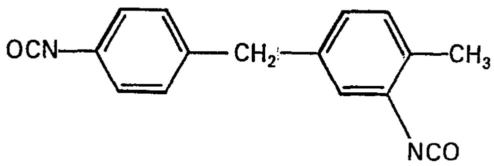

et 10 à 40% en poids, par rapport au mélange total, de diisocyanates de formule:

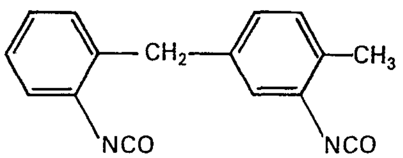

ainsi que 0 à 30% en poids, par rapport au mélange total, d'autres isomères de diisocyanatodiphénylméthane à substituants méthyle, le reste $R_2$ dans chacune des deux formules représentant un groupe méthyle et les deux autres restes représentant dans chaque cas de l'hydrogène, caractérisé en ce que:

a) on fait réagir des mélanges techniques d'isomères de chlorure de nitrobenzyle obtenus par nitration du chlorure de 4-méthylbenzyle, contenant 10 à 25% en poids, par rapport au mélange total, de chlorure de 2-nitro-4-méthylbenzyle et 75 à 90% en poids, par rapport au mélange total, de chlorure de 3-nitro-4-méthylbenzyle, en présence de catalyseurs de Friedel-Crafts avec du benzène pour former un produit de condensation de Friedel-Crafts contenant principalement les composés mononitrés de formules:

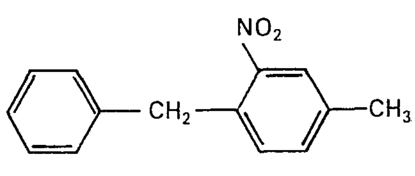

et comme produit principal

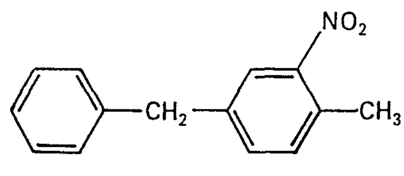

on élimine le catalyseur du produit de condensation,

b) on soumet le produit de condensation obtenu conformément à a) à une réaction de nitration avec formation d'un mélange de composés dinitrés aromatiques contenant principalement des composés nitrés de formules:

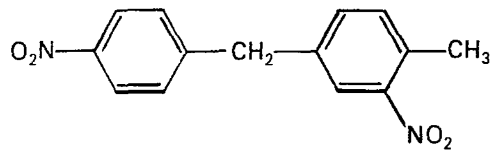

et

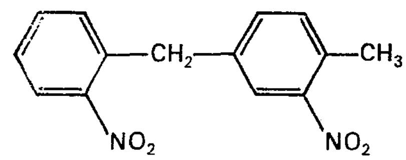

c) on transforme les composés nitrés obtenus conformément à b) par hydrogénation ou réduction des groupes nitro en les diamines aromatiques correspondantes,

d) on transforme les diamines obtenues conformément à c) par phosgénation en les diisocyanates correspondants, et, le cas échéant,

e) on isole par distillation du mélange, obtenu conformément à c), de composés aminés aromatiques avant la phosgénation et/ou du mélange, obtenu conformément à d), de polyisocyanates, le mélange d'isomères contenant 30 à 60% en poids, par rapport au mélange total, d'isomères 3,4' et 10 à 40% en poids, par rapport au mélange total, d'isomères 3,2', ainsi que 0 à 30% en poids, par rapport au mélange total, d'autres isomères de diisocyanatodiphénylméthane portant des substituants méthyle, sous une forme débarrassée des sous-produits.

6. Procédé de production de diisocyanates de formule:

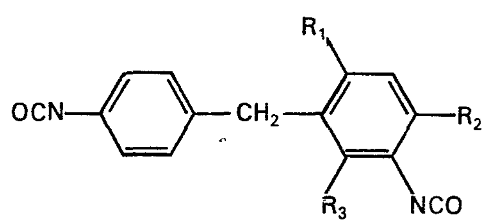

présents en mélange avec jusqu'à 40% en poids, par rapport au mélange total, de diisocyanates de formule:

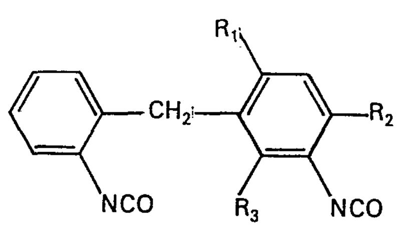

et avec jusqu'à 30% en poids, par rapport au mélange total, d'autres isomères de diisocyanatodiphénylméthane à substituants méthyle, l'un des restes $R_1$, $R_2$ ou $R_3$ dans chacune des deux formules désignant un reste méthyle et les deux autres restes désignant dans chaque cas de l'hydrogène, caractérisé en ce que

a) on fait réagir un halogénure de benzyle en présence de catalyseurs de Friedel-Crafts ou l'alcool benzylique en présence de catalyseurs acides avec le toluène pour former un produit de condensation principalement composé d'hydrocarbures de formule:

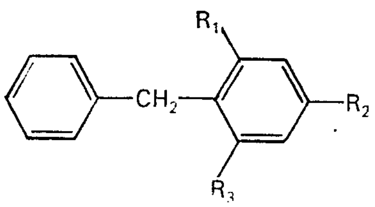

b) on soumet à une dinitration le mélange d'isomères de méthyldiphénylméthane de la formule mentionnée en dernier lieu, obtenu par distillation sous la forme pure à partir du (en outre) produit de condensation obtenu conformément à a),

c) on transforme les composés dinitrés obtenus conformément à b) par hydrogénation ou réduction des groupes nitro en les diamines correspondantes,

d) on transforme les diamines obtenues conformément à c) par phosgénation en les diisocyanates correspondants, et, le cas échéant,

e) on isole des diamines obtenues conformément à c) avant la phosgénation et/ou des mélanges de diisocyanates obtenus conformément à d), l'isomère 3,4' correspondant se présentant en mélange avec jusqu'à 40% en poids d'isomères 2', 3 et avec jusqu'à 30% en poids, par rapport au mélange total, d'autres isomères de diisocyanatodiphénylméthane à substituants méthyle, sous une forme débarrassée des sous-produits.

7. Procédé de production de 3,4'-diisocyanato-4-méthyldiphénylméthane se présentent en mélange avec jusqu'à 40% en poids, par rapport au mélange total, de 2',3-diisocyanato-4-méthyldiphénylméthane et jusqu'à 30% en poids, par rapport au mélange total, d'autres isomères de diisocyanatodiphénylméthane à substituants méthyle, caractérisé en ce que

a) on fait réagir un halogénure de p-méthylbenzyle en presence de catalyseurs de Friedel-Crafts avec du benzène pour former un mélange d'hydrocarbures composé de 4-méthyl-diphénylméthane et de condensats de plus haut poids moléculaire,

b) on effectue la dinitration du 4-méthyldiphénylméthane obtenu par distillation sous la forme pure à partir du mélange d'hydrocarbures obtenu conformément à a),

c) on transforme le composé dinitré obtenu conformément à b) par hydrogénation ou réduction des groupes nitro en le composé diamino aromatique correspondant,

d) on transforme les diamines obtenues conformément à c) par phosgénation en les diisocyanates correspondants, et, le cas échéant,

e) on isole des diamines obtenues conformément à c) avant la réaction de phosgénation et/ou des diisocyanates obtenus conformément à d), les isomères 3,4' correspondants en mélange avec jusqu'à 30% en poids, par rapport au mélange total, d'isomères 2',3 et jusqu'à 30% en poids, par rapport au mélange total, d'autres isomères de diisocyanatodiphénylméthane à substituants méthyle, sous une forme débarrassée des sous-produits.

8. Utilisation des diisocyanates et mélanges de diisocyanates suivant les revendications 1 à 7 comme composant isocyanate dans la production de matières plastiques du type polyuréthanne par le procédé de polyaddition d'un isocyanate.